# EUROPÄISCHE PATENTSCHRIFT

(11) **EP 1 370 612 B1**
(45) Veröffentlichungstag und Bekanntmachung des Hinweises auf die Patenterteilung: **22.09.2004**
(21) Anmeldenummer: 02718131.2
(22) Anmeldetag: 14.02.2002
(51) Int. Cl.: C09B 5/62, C08K 5/3437, C09D 11/00

(54) **THERMOCHROME RYLENFARBSTOFFE**
THERMOCHROMIC RYLENE DYES
COLORANT DE RYLENE THERMOCHROMES

(30) Priorität: 22.02.2001 DE 10108601
(43) Veröffentlichungstag der Anmeldung: 17.12.2003
(73) Patentinhaber: BASF AKTIENGESELLSCHAFT, 67056 Ludwigshafen (DE); Max-Planck-Gesellschaft zur Förderung der Wissenschaften e.V., 80539 München (DE)
(72) Erfinder: BÖHM, Arno, 68305 Mannheim (DE); KRIEGER, Matthias, 79539 Lörrach (DE); BECKER, Stefan, 68167 Mannheim (DE); MÜLLEN, Klaus, 50939 Köln (DE)
(86) Internationale Anmeldenummer: PCT/EP2002/001573
(87) Internationale Veröffentlichungsnummer: WO 2002/068538

(56) Entgegenhaltungen:
- EP-A- 0 657 436
- WO-A-00/52099
- WO-A-01/16109
- WO-A-96/22332
- DE-A- 19 512 773
- DE-A- 19 848 555
- GEERTS Y ET AL: "QUATERRYLENEBIS(DICARBOXIMIDE)S: NEAR INFRARED ABSORBING AND EMITTING DYES" JOURNAL OF MATERIALS CHEMISTRY, THE ROYAL SOCIETY OF CHEMISTRY, CAMBRIDGE, GB, Bd. 8, Nr. 11, November 1998 (1998-11), Seiten 2357-2369, XP000803180 ISSN: 0959-9428

## Beschreibung

Die vorliegende Erfindung betrifft neue Rylenfarbstoffe der allgemeinen Formel I 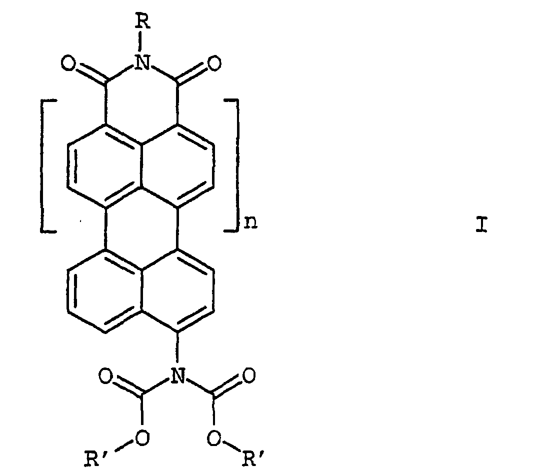 in der die Variablen folgende Bedeutung haben:
- R: Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
Aryl oder Hetaryl, das jeweils durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy und/ oder Cyano substituiert sein kann, ein- oder mehrfach substituiert sein kann;
- R': C₂-C₃₀-Alkyl, dessen Kohlenstoffkette durch eine oder mehrere Gruppierungen -O- und/oder -CO- unterbrochen sein kann und das durch Cyano, C₁-C₆-Alkoxy, C₅-C₈-Cycloalkyl, dessen Kohlenstoffgerüst durch eine oder mehrere Gruppierungen -O-, - S- und/oder -NR¹- unterbrochen sein kann und das durch C₁-C₆-Alkyl substituiert sein kann, Aryl, das durch C₁-C₁₈-Alkyl oder C₁-C₆-Alkoxy substituiert sein kann, und/ oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
Methyl, das durch Aryl, Hetaryl und/oder C₅-C₈-Cycloalkyl, das jeweils durch C₁-C₁₈-Alkyl oder C₁-C₆-Alkoxy substituiert sein kann, ein- oder zweifach substituiert ist;
C₅-C₈-Cycloalkyl, dessen Kohlenstoffgerüst durch eine oder mehrere Gruppierungen -O-, -S- und/oder -NR¹- unterbrochen sein kann und das durch C₁-C₆-Alkyl ein- oder mehrfach substituiert sein kann;
- R¹: Wasserstoff oder C₁-C₆-Alkyl;
- R²: Wasserstoff; C₁-C₁₈-Alkyl; Aryl oder Hetaryl, das jeweils durch C₁-C₆-Alkyl, C₁-C₆-Alkoxy und/oder Cyano substituiert sein kann;
- n: 2 oder 3,
sowie die Herstellung dieser Farbstoffe und ihre Verwendung zur Einfärbung von hochmolekularen organischen und anorganischen Materialien.

Außerdem betrifft die Erfindung neue Aminorylen-3,4-dicarbonsäureimide der allgemeinen Formel IV 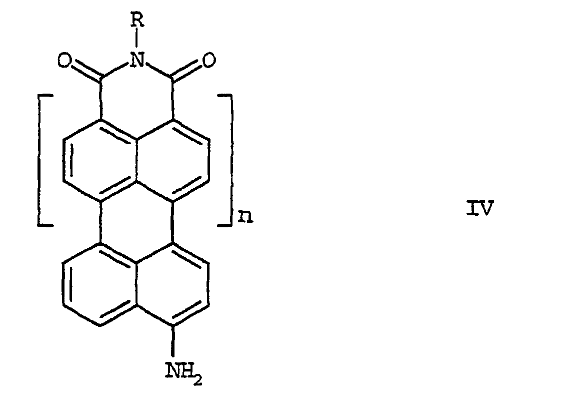 als Zwischenprodukte für die Rylenfarbstoffe (I).

Am Imidstickstoffatom substituierte Perylen-3,4-dicarbonsäureimide, unsubstituiertes Perylen-3,4-dicarbonsäureimid und am Perylengerüst substituierte Perylen-3,4-dicarbonsäureimide eignen sich nicht nur als Pigmentvorstufen, sondern werden auch selbst vorteilhaft als Pigmente und Fluoreszenzfarbstoffe eingesetzt. Die bislang bekannten am Perylengerüst substituierten Perylen-3,4-dicarbonsäureimide sind in 1,6-, 1,7-, 1,6,9-, 1,7,9-und 1,6,7,12-Stellung sowie auch nur in 9-Stellung substituiert. In 9-Stellung trägt das Perylengerüst dabei jeweils ein Halogenatom, insbesondere Bromatom, (WO-A-96/22331, EP-A-596 292 und WO-A-97/22607 und die dort zitierte Literatur sowie Dyes and Pigments 16, Seite 19-25 (1991)). In der EP-A-657 436 sowie Liebigs Annalen 1995, Seite 1229-1244, ist außerdem ein N-(1-Hexylheptyl)-9-aminoperylen-3,4-dicarbonsäureimid beschrieben, das durch Nitrierung des entsprechenden N-substituierten Perylen-3,4-dicarbonsäureimids mit Distickstofftetroxid und anschließende Reduktion mit metallischem Eisen in Gegenwart von Salzsäure hergestellt wird. Dieses Verfahren ist jedoch auf Perylen-3,4-dicarbonsäureimide beschränkt, die unsubstituierte Alkylgruppen am Imidstickstoffatom tragen, und liefert ausschließlich schwer zu reinigende Isomerengemische (1- und 9-Isomer) in niedrigen Ausbeuten. Entsprechende N-substituierte 4-Aminonaphthalin-1,8-dicarbonsäureimide sind aus Yuki Gosei Kagaku Kyokaishi 12, Seite 504-508 (1956) (s. Chemical Abstracts 51:8052a (1957)) bekannt.

In der EP-A-648 817 sind imidgruppenhaltige Fluoreszenzfarbstoffe beschrieben, deren Imidstickstoffatom zur reversiblen Solubilisierung in eine Carbamatfunktion überführt ist, die den Farbstoff im Anwendungsmedium löslich macht und thermisch wieder gespalten werden kann. Unter anderem wird hier auch unsubstituiertes Perylen-3,4-dicarbonsäureimid, dessen NH-Funktion entsprechend umgesetzt wird, als Fluoreszenzfarbstoff aufgeführt. Da die Solubilisierung über das Imidstickstoffatom erfolgt, besteht keine Möglichkeit, den Farbstoff über spezielle Substitution am Stickstoffatom zu modifizieren. Außerdem verändert sich der Farbton des Farbstoffs bei der thermischen Abspaltung der Alkoxycarbonyl-Schutzgruppe nicht, der Farbstoff ist nicht thermochrom.

In der nicht vorveröffentlichten WO-A-01/16109 sind kürzere Homologe der erfindungsgemäßen Rylenfarbstoffe auf Basis von Naphthalin- und Perylen-3,4-dicarbonsäurederivaten beschrieben. Diese decken jedoch nur den kürzerwelligen Farbraum ab.

Der Erfindung lag die Aufgabe zugrunde, weitere Farbstoffe mit vorteilhaften Anwendungseigenschaften, die insbesondere auch nicht nur gut in das jeweilige Anwendungsmedium einarbeitbar und an dieses Medium anpaßbar, sondern auch thermochrom sind und im roten und infraroten Bereich des elektromagnetischen Spektrums absorbieren, bereitzustellen.

Demgemäß wurden die eingangs definierten Rylenfarbstoffe der Formel I gefunden.

Bevorzugte Rylenfarbstoffe sind den Unteransprüchen zu entnehmen.

Weiterhin wurde ein Verfahren zur Herstellung der Rylenfarbstoffe I gefunden, welches dadurch gekennzeichnet ist, daß man
a) ein Bromrylen-3,4-dicarbonsäureimid der allgemeinen Formel II 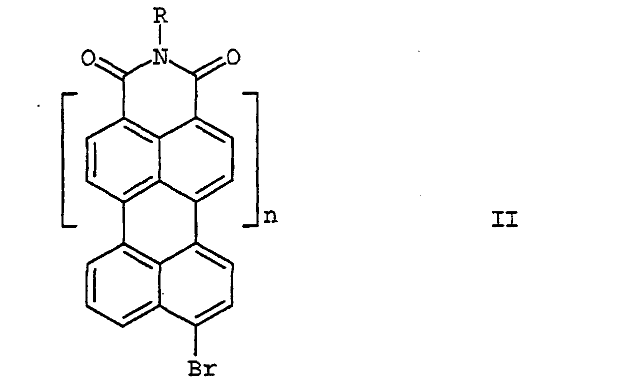 in Gegenwart eines aprotischen organischen Lösungsmittels, eines Übergangsmetallkatalysatorsystems und einer Base in einer Aryl-N-Kupplungsreaktion mit einem Benzophenonimin der allgemeinen Formel III 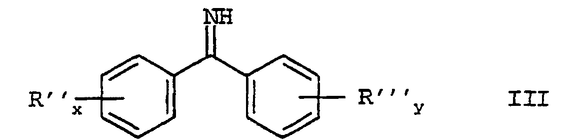 in der die Variablen
   - R'', R''': unabhängig voneinander Wasserstoff, C₁-C₆-Alkyl oder C₁-C₆-Alkoxy und
   - x, y: unabhängig voneinander eine ganze Zahl von 1 bis 3 bedeuten,
   umsetzt,
b) das gebildete Ketimin in Gegenwart einer Säure und eines polaren, aprotischen Lösungsmittels zu einem Aminorylen-3, 4-dicarbonsäureimid der allgemeinen Formel IV 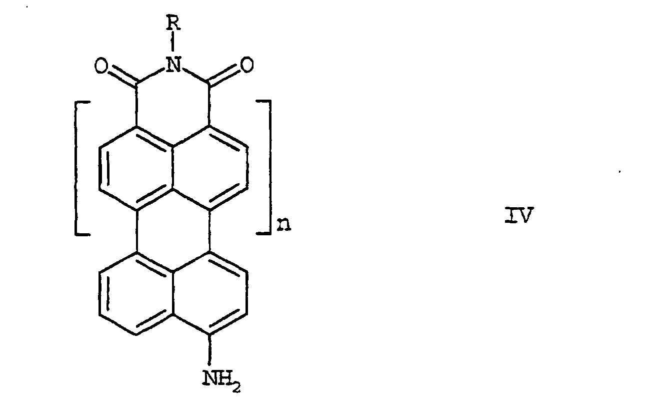 hydrolysiert und
c) dieses anschließend in Gegenwart eines polaren, aprotischen Lösungsmittels und einer Base mit einem Dicarbonat der allgemeinen Formel V 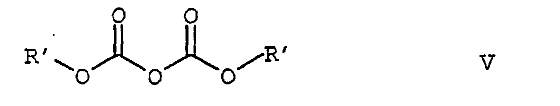 zu dem Rylenfarbstoff I umsetzt.

Außerdem wurde ein Verfahren zur Herstellung der Aminorylen-3,4-dicarbonsäureimide IV gefunden, welches dadurch gekennzeichnet ist, daß man a) ein Bromrylen-3,4-dicarbonsäureimid II in Gegenwart eines aprotischen organischen Lösungsmittels, eines Übergangsmetallkatalysatorsystems und einer Base in einer Aryl-N-Kupplungsreaktion mit einem Benzophenonimin III zu dem entsprechenden Ketimin umsetzt und b) dieses anschließend in Gegenwart einer Säure und eines polaren, aprotischen Lösungsmittels hydrolysiert.

Zudem wurden die Aminorylen-3,4-dicarbonsäureimide der allgemeinen Formel IV als Zwischenprodukte für die Rylenfarbstoffe I gefunden.

Nicht zuletzt wurde die Verwendung der Rylenfarbstoffe I zur Einfärbung von hochmolekularen organischen und anorganischen Materialien gefunden.

Alle in den Formeln I bis V auftretenden Alkylgruppen können sowohl geradkettig als auch verzweigt sein. Wenn die Alkylgruppen substituiert sind, tragen sie in der Regel 1 oder 2 Substituenten. Aromatische Reste, die substituiert sind, können im allgemeinen bis zu 3, bevorzugt 1 oder 2, der genannten Substituenten aufweisen. Bevorzugte Arylreste sind dabei Naphthyl und insbesondere Phenyl.

Als Beispiele für geeignete Reste R, R', R", R''', R¹ und R² (bzw. für deren Substituenten) seien im einzelnen genannt:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den bei der Oxosynthese erhaltenen Alkoholen);
2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2- und 3-Propoxypropyl, 2- und 3- Butoxypropyl, 2- und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
2-Methylthioethyl, 2-Ethylthioethyl, 2-Propylthioethyl, 2-Isopropylthioethyl, 2-Butylthioethyl, 2- und 3-Methylthiopropyl, 2- und 3-Ethylthiopropyl, 2- und 3-Propylthiopropyl, 2- und 3-Butylthiopropyl, 2- und 4-Methylthiobutyl, 2- und 4-Ethylthiobutyl, 2- und 4-Propylthiobutyl, 3,6-Dithiaheptyl, 3,6-Dithiaoctyl, 4,8-Dithianonyl, 3,7-Dithiaoctyl, 3,7-Dithianonyl, 4,7-Dithiaoctyl, 4,7-Dithianonyl, 2- und 4-Butylthiobutyl, 4,8-Dithiadecyl, 3,6,9-Trithiadecyl, 3,6,9-Trithiaundecyl, 3,6,9-Trithiadodecyl, 3,6,9,12-Tetrathiatridecyl und 3,6,9,12-Tetrathiatetradecyl;
2-Monomethyl- und 2-Monoethylaminoethyl, 2-Dimethylaminoethyl, 2- und 3-Dimethylaminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Diazaoctyl, 3,6-Dimethyl-3,6-diazaoctyl, 9-Methyl-3,6,9-triazadecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
Propan-2-on-1-yl, Butan-3-on-1-yl, Butan-3-on-2-yl und 2-Ethylpentan-3-on-1-yl;
2-Methylsulfonylethyl, 2-Ethylsulfonylethyl, 2-Propylsulfonylethyl, 2-Isopropylsulfonylethyl, 2-Buthylsulfonylethyl, 2- und 3-Methylsulfonylpropyl, 2- und 3-Ethylsulfonylpropyl, 2- und 3-Propylsulfonylpropyl, 2- und 3-Butylsulfonylpropyl, 2- und 4-Methylsulfonylbutyl, 2- und 4-Ethylsulfonylbutyl, 2- und 4-Propylsulfonylbutyl und 4-Butylsulfonylbutyl;
Cyanomethyl, 2-Cyanoethyl, 3-Cyanopropyl, 2-Methyl-3-ethyl-3-cyanopropyl, 7-Cyano-7-ethylheptyl und 4-Methyl-7-methyl-7-cyanoheptyl;
Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;
Carbamoyl, Methylaminocarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl, Decylaminocarbonyl und Phenylaminocarbonyl;
Formylamino, Acetylamino, Propionylamino und Benzoylamino;
Phenylazo, 2-Naphthylazo, 2-Pyridylazo und 2-Pyrimidylazo;
Phenyl, 2-Naphthyl, 2- und 3-Pyrryl, 2-, 3- und 4-Pyridyl, 2-, 4-und 5-Pyrimidyl, 3-, 4- und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothiazolyl, 5-Benzothiadiazolyl, 2- und 5-Benzimidazolyl und 1- und 5-Isochinolyl;
2-, 3- und 4-Methylphenyl, 2,4-, 2,5-, 3,5- und 2,6-Dimethylphenyl, 2,4,6-Trimethylphenyl, 2-, 3- und 4-Ethylphenyl, 2,4-, 2,5-, 3,5- und 2,6-Diethylphenyl, 2,4,6-Triethylphenyl, 2-, 3- und 4-Propylphenyl, 2,4-, 2,5-, 3,5- und 2,6-Dipropylphenyl, 2,4,6-Tripropylphenyl, 2-, 3- und 4-Isopropylphenyl, 2,4-, 2,5-, 3,5- und 2,6-Diisopropylphenyl, 2,4,6-Triisopropylphenyl, 2-, 3- und 4-Butylphenyl, 2,4-, 2,5-, 3,5- und 2,6-Dibutylphenyl, 2,4,6-Tributylphenyl, 2-, 3- und 4-Isobutylphenyl, 2,4-, 2,5-, 3,5- und 2,6-Diisobutylphenyl, 2,4,6-Triisobutylphenyl, 2-, 3- und 4-sec.-Butylphenyl, 2,4-, 2,5-, 3,5- und 2,6-Di-sec.-butylphenyl und 2,4,6-Tri-sec.-butylphenyl, 2-, 3- und 4-tert.-Butylphenyl, 2,4-, 2,5-, 3,5- und 2,6-Di-tert.-butylphenyl, 2,4,6-Tritert.-butylphenyl; 2-, 3- und 4-Methoxyphenyl, 2,4-, 2,5-, 3,5- und 2,6-Dimethoxyphenyl, 2,4,6-Trimethoxyphenyl, 2-, 3- und 4-Ethoxyphenyl, 2,4-, 2,5-, 3,5- und 2,6-Diethoxyphenyl, 2,4,6-Triethoxyphenyl, 2-, 3- und 4-Propoxyphenyl, 2,4-, 2,5-, 3,5- und 2,6-Dipropoxyphenyl, 2-, 3- und 4-Isopropoxyphenyl, 2,4-, 2,5-, 3,5- und 2,6-Diisopropoxyphenyl und 2-, 3- und 4-Butoxyphenyl; 2-, 3- und 4-Cyanophenyl; 3- und 4-Carboxyphenyl; 3- und 4-Carboxamidophenyl, 3- und 4-N-Methylcarboxamidophenyl und 3- und 4-N-Ethylcarboxamidophenyl; 3- und 4-Acetylaminophenyl, 3- und 4-Propionylaminophenyl und 3- und 4-Butyrylaminophenyl; 3- und 4-N-Phenylaminophenyl, 3- und 4-N-(o-Tolyl)aminophenyl, 3- und 4-N-(m-Tolyl)aminophenyl und 3- und 4-N-(p-Tolyl)aminophenyl; 3- und 4-(2-Pyridyl)aminophenyl, 3- und 4-(3-Pyridyl)aminophenyl, 3- und 4-(4-Pyridyl)aminophenyl, 3- und 4-(2-Pyrimidyl)aminophenyl und 4-(4-Pyrimidyl)aminophenyl;
4-Phenylazophenyl, 4-(1-Napthylazo)phenyl, 4-(2-Naphthylazo)phenyl, 4-(4-Naphthylazo)phenyl, 4-(2-Pyridylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo)phenyl;
Cyclopentyl, 2- und 3-Methylcyclopentyl, 2- und 3-Ethylcyclopentyl, Cyclohexyl, 2-, 3- und 4-Methylcyclohexyl, 2-, 3- und 4-Ethylcyclbhexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methylcycloheptyl, 2-, 3- und 4-Ethylcycloheptyl, 3- und 4-Propylcycloheptyl, 3- und 4-Isopropylcycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethylcyclooctyl, 3-, 4- und 5-Propylcyclooctyl, 2-Dioxanyl, 4-Morpholinyl, 2- und 3-Tetrahydrofuryl, 1-, 2- und 3-Pyrrolidinyl und 1-, 2-, 3- und 4-Piperidyl.

Die Herstellung der Rylenfarbstoffe I kann vorteilhaft nach dem erfindungsgemäßen mehrstufigen Verfahren erfolgen, bei dem in Schritt a) ein Bromrylen-3,4-dicarbonsäureimid II mit einem Benzophenonimin III zu einem Ketimin umgesetzt wird, in Schritt b) das Ketimin sauer zu dem Aminorylen-3,4-dicarbonsäureimid IV hydrolysiert wird, welches anschließend in Schritt c) mit einem Dicarbonat V zum Rylenfarbstoff I umgesetzt wird.

Die in Schritt a) als Edukte eingesetzten in peri-Position bromierten Terrylen- bzw. Quaterrylen-3,4-dicarbonsäureimide II sind aus der älteren deutschen Patentanmeldung 101 08 156.1 bekannt und können nach dem dort beschriebenen, folgenden dreistufigen Verfahren hergestellt werden:
a') einseitige alkalische Verseifung eines asymmetrischen Rylentetracarbonsäurediimids der Formel VI 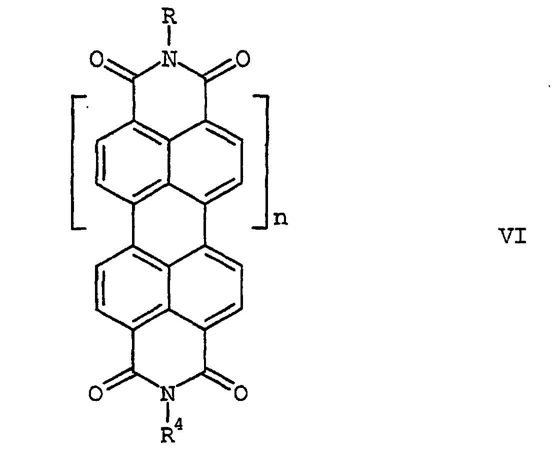 in der R⁴ C₅-C₈-Cycloalkyl, dessen Kohlenstoffgerüst durch eine oder mehrere Gruppierungen -O-, -S- und/oder -NR²- unterbrochen sein kann und das durch C₁-C₆-Alkyl ein- oder mehrfach substituiert sein kann, bedeutet,
   in Gegenwart eines polaren organischen Lösungsmittels,
b') einseitige Decarboxylierung des in Schritt a') gebildeteten Rylentetracarbonsäuremonoimidmonoanhydrids der Formel VII 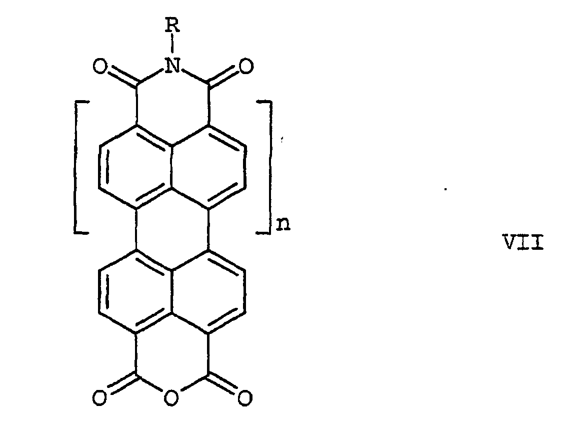 in Gegenwart einer tertiären stickstoffbasischen Verbindung und eines Übergangsmetallkatalysators und
c') Umsetzung des in peri-Position unsubstituierten Rylen-3,4-dicarbonsäureimids der Formel VIII 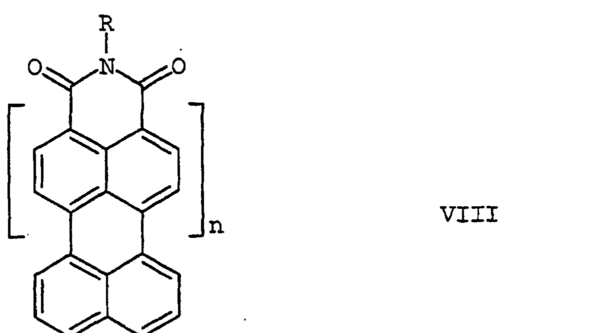 mit elementarem Brom.

Für Schritt a') dieses Verfahrens geeignete polare Lösungsmittel sind insbesondere verzweigte C₃-C₆-Alkohole, wie Isopropanol, tert.-Butanol und 2-Methyl-2-butanol.

Im allgemeinen kommen 40 bis 200 g Lösungsmittel je g VI zum Einsatz.

Als Basen eignen sich anorganische Basen, insbesondere Alkali- und Erdalkalimetallhydroxide, z.B. Natriumhydroxid und Kaliumhydroxid, die vorzugsweise in Form von wäßrigen Lösungen bzw. Suspensionen (in der Regel 50 bis 80 gew.-%ig) verwendet werden, und organische Basen, insbesondere Alkali- und Erdalkalimetallalkoxide, wobei Natrium- und Kaliumalkoxide, wie Natriummethylat, Kaliummethylat, Kaliumisopropylat und Kalium-tert.-butylat bevorzugt sind, die üblicherweise in wasserfreier Form eingesetzt werden.

In der Regel werden 5 bis 50 Äquivalente Base, bezogen auf VI, benötigt.

Die Reaktionstemperatur beträgt im allgemeinen 50 bis 120°C, vorzugsweise 60 bis 100°C.

Die Verseifung ist üblicherweise in 10 bis 40 h abgeschlossen.

In Schritt b') dieses Verfahrens werden die Rylentetracarbonsäuremonoimidmonoanhydride VII in Gegenwart einer tertiären stickstoffbasischen Verbindung als Lösungsmittel und eines Übergangsmetallkatalysators einseitig decarboxyliert.

Geeignete Lösungsmittel sind insbesondere hochsiedende Stickstoffbasen, z.B. cyclische Amide, wie N-Methylpyrrolidon, und aromatische Heterocyclen, wie Chinolin, Isochinolin und Chinaldin.

Übliche Lösungsmittelmengen betragen 20 bis 150 g je g VII.

Als Katalysatoren eignen sich insbesondere die Übergangsmetalle Kupfer und Zink sowie besonders auch deren anorganische und organische Salze, die vorzugsweise in wasserfreier Form eingesetzt werden.

Beispiele für bevorzugte Salze sind Kupfer(I)oxid, Kupfer(II)oxid, Kupfer (I) chlorid, Kupfer(II)acetat, Zinkacetat und Zinkpropionat, wobei Kupfer(I)oxid und Zinkacetat besonders bevorzugt sind.

Selbstverständlich kann man auch Mischungen der genannten Katalysatoren verwenden.

In der Regel kommen 50 bis 90 mol-% Katalysator, bezogen auf VII, zum Einsatz.

Die Reaktionstemperatur liegt im allgemeinen bei 100 bis 250°C, insbesondere 160 bis 200°C. Es empfiehlt sich, unter Verwendung einer Schutzgasatmosphäre (z.B. Stickstoff) zu arbeiten.

Üblicherweise ist die Decarboxylierung in 3 bis 20 h beendet.

Schritt c') dieses Verfahrens, die regioselektive Bromierung des Rylen-3,4-dicarbonsäureimids VIII, wird vorzugsweise in einer aliphatischen Monocarbonsäure, insbesondere einer C₁-C₄-Carbonsäure, wie Ameisensäure, Essigsäure, Propionsäure, Buttersäure oder deren Mischungen, oder in einem halogenierten, aliphatischen oder aromatischen Lösungsmittel, wie Methylenchlorid, Chloroform oder Chlorbenzol, durchgeführt.

Üblicherweise werden 5 bis 30 g, vorzugsgweise 15 bis 25 g, Lösungsmittel je g zu bromierendes VIII eingesetzt.

In der Regel ist die Anwesenheit eines Halogenierungskatalysators nicht erforderlich. Will man jedoch die Bromierungsreaktion beschleunigen (etwa um den Faktor 1,5 bis 2), so empfiehlt es sich, elementares Iod, bevorzugt in einer Menge von 1 bis 5 mol-%, bezogen auf VIII, zuzusetzen.

Im allgemeinen liegt das Molverhältnis von Brom zu VIII bei etwa 1 : 1 bis 5 : 1, vorzugsweise bei 3 : 1 bis 4 : 1.

Die Reaktionstemperatur beträgt in der Regel 0 bis 70°C, insbesondere 10 bis 40°C.

In Abhängigkeit von der Reaktivität des Substrats VIII und der An- oder Abwesenheit von Iod ist die Bromierung üblicherweise in 2 bis 12 h beendet.

Schritt a) des vorliegenden, erfindungsgemäßen Verfahrens, die Umsetzung des Bromrylen-3,4-dicarbonsäureimids II mit einem Benzophenon III in einer Aryl-N-Kupplungsreaktion zu einem Ketimin, wird in Gegenwart eines aprotischen organischen Lösungsmittels, eines Übergangsmetallkatalysatorsystems und einer Base durchgeführt.

Als Benzophenonimin III eignen sich für die Ketiminbildung insbesondere Benzophenonimin, 4,4'-Dimethyl- und 4,4'-Diethylbenzophenonimin, 2,2',4,4'-Tetramethylbenzophenonimin und 4,4'-Dimethoxy- und 4,4'-Diethoxybenzophenonimin, wobei Benzophenonimin selbst bevorzugt ist.

In der Regel werden 1 bis 4 mol, vorzugsweise 1,5 bis 2,5 mol, III je mol II eingesetzt.

Als aprotisches organisches Lösungsmittel sind wasserfreie inerte aromatische Lösungsmittel, wie Benzol und seine Alkylierungsprodukte, z.B. Toluol und o-, m- und p-Xylol, und Mischungen dieser Verbindungen, besonders geeignet.

Die Lösungsmittelmenge beträgt üblicherweise 50 bis 300 kg, vorzugsweise 100 bis 200 kg, je kg II.

Als Übergangsmetallkatalysator eignen sich insbesondere Palladiumverbindungen, wobei Palladium(0)- und Palladium(II)-Komplexe, wie Tris(dibenzylidenaceton)dipalladium(0), Dichloro[1,1'-bis(diphenylphosphino)ferrocen]palladium(II) und Dichloro(1,5-cyclooctadien)palladium(II), und Palladium(II)acetat als bevorzugte Beispiele zu nennen sind.

Üblicherweise wird der Übergangsmetallkatalysator in einer Menge von 0,5 bis 5 mol-%, vor allem 1 bis 3 mol-%, bezogen auf II, eingesetzt.

Vorzugsweise kommt zusätzlich ein Cokatalysator auf Phosphinbasis zum Einsatz. Bevorzugte Beispiele für diesen Cokatalysator sind zweizähnige Phosphinliganden, wie racemisches 2,2'-Bis(diphenylphosphino)-1,1'-binaphthyl, 1,1'-Bis(diphenylphosphino)ferrocen, 1,1'-Bis(di-o-tolylphosphino)ferrocen, 1,1'-Bis(di-p-methoxyphenylphosphino)ferrocen und 2,2'-Bis(di-o-tolylphosphino)-diphenylether, und als einzähnige Phosphinliganden wirkende Phosphine, wie Tri-o-tolylphosphin, Tri-tert.-butylphosphin und Triphenylphosphin.

Geeignete Cokatalysatormengen betragen in der Regel 2 bis 5 mol-%, vorzugsweise 1 bis 3 mol-%, bezogen auf den Übergangsmetallkatalysator.

Als Base eignen sich besonders Alkalimetallamide, vor allem Alkalimetalldi(C₃-C₆-alkyl)amide, und Alkalimetallalkoholate, vor allem die Alkalimetallsalze sekundärer und tertiärer aliphatischer (C₃-C₆-)Alkohole. Bevorzugte Bespiele für diese Basen sind: Lithiumdiisopropylamid, Natriumdiisopropylamid und Kaliumdiisopropylamid sowie Lithiumisopropanolat, Natriumisopropanolat, Kaliumisopropanolat, Lithium-tert.-butanolat, Natrium-tert.-butanolat und Kalium-tert.-butanolat, wobei Natrium-tert.-butanolat und Kalium-tert.-butanolat besonders bevorzugt sind.

Im allgemeinen wird eine zum Benzophenonimin III äquimolare Menge Base eingesetzt.

Die Reaktionstemperatur liegt üblicherweise bei 50 bis 140°C, bevorzugt bei 70 bis 120°C.

Die Reaktionszeit beträgt in Abhängigkeit von der Reaktivität des bromierten Rylen-3,4-dicarbonsäureimids II und der eingesetzten Katalysatormenge im allgemeinen 10 bis 24 h.

Verfahrenstechnisch geht man in Schritt a) zweckmäßigerweise wie folgt vor:

Man legt Lösungsmittel, Katalysator und Cokatalysator in einer Schutzgasatmosphäre vor, setzt unter Rühren nacheinander das Bromrylen-3,4-dicarbonsäureimid II, das Benzophenonimin III und Base zu und erhitzt 10 bis 24 h unter Schutzgas auf die gewünschte Reaktionstemperatur. Nach Abkühlen auf Raumtemperatur filtriert man die festen Bestandteile aus dem Reaktionsgemisch ab und destilliert das Lösungsmittel unter vermindertem Druck ab.

Die Reinheit des so hergestellten Ketimins reicht im allgemeinen für die Weiterverarbeitung aus. Gegebenenfalls kann das Rohprodukt durch Umfällen aus einem Gemisch aus Chloroform oder Methylenchlorid und Petrolether oder über Säulenchromatographie an Kieselgel mit Chloroform als Eluens weiter aufgereinigt werden.

Schritt b) des erfindungsgemäßen Verfahrens, die Hydrolyse des Ketimins zum Aminorylen-3,4-dicarbonsäureimid IV, wird in Gegenwart eines polaren, aprotischen Lösungsmittels vorgenommen. Bevorzugte Lösungsmittel sind aliphatische Ether, wobei acyclische Ether, wie insbesondere Di(C₂-C₄-alkyl)ether und C₂-C₃-Alkylenglykoldi-C₁-C₂-alkylether, und cyclische Ether geeignet sind. Beispielhaft seien folgende besonders bevorzugte Ether genannt: Diethylether, Dipropylether, Dibutylether, Ethylenglykoldimethylund -diethylether, Tetrahydrofuran und Dioxan.

In der Regel kommen 80 bis 300 kg, vorzugsweise 100 bis 200 kg, Lösungsmittel je kg Ketimin zum Einsatz.

Zur Hydrolyse wird vorzugsweise eine anorganische Säure, wie Salzsäure, Schwefelsäure, Phosphorsäure und Salpetersäure, verwendet.

Üblicherweise werden 3 bis 6 kg einer 2 bis 4 normalen wäßrigen Lösung der Säure je kg Ketimin eingesetzt.

Die Reaktionstemperatur beträgt im allgemeinen 10 bis 60°C, vorzugsweise 20 bis 40°C.

Die Hydrolyse ist in der Regel in 0,5 bis 3 h beendet.

Verfahrenstechnisch geht man in Schritt b) zweckmäßigerweise wie folgt vor:

Man löst das Ketimin unter Rühren im Lösungsmittel, bringt das Gemisch auf die gewünschte Reaktionstemperatur, gibt die wäßrige Säure zu und rührt 0,5 bis 3 h bei dieser Temperatur. Anschließend neutralisiert man die verbliebene Säure, z.B. mit konzentriertem wäßrigem Ammoniak, und destilliert das Lösungsmittel im Vakuum ab.

Zur weiteren Aufbereitung des Reaktionsprodukts kann man dann wie folgt verfahren:

Man suspendiert den Rückstand in einem Überschuß an verdünnter wäßriger Base (z.B. Ammoniakwasser), filtriert ab, rührt das Filtergut gegebenenfalls mehrfach in der 30 bis 50-fachen Menge an heißer wäßriger Base (etwa halbkonzentriertem wäßrigem Ammoniak) aus, filtriert erneut, wäscht mit Wasser neutral und trocknet das Filtergut im Vakuum bei 100°C. Zur Entfernung von Benzophenon und weiteren organischen Verunreinigungen extrahiert man das getrocknete Rohprodukt anschließend mit Petrolether.

Schritt c) des erfindungsgemäßen Verfahrens, die Umsetzung des Aminorylendicarbonsäureimids IV mit einem Dicarbonat V zum Rylenfarbstoff I, wird in Gegenwart eines polaren, aprotischen Lösungsmittels basenkatalysiert durchgeführt.

Besonders bevorzugte Dicarbonate V sind Dialkyldicarbonate, vor allem Di-(C₂-C₈-alkyl)dicarbonate, wie Diethyl-, Dipropyl-, Diisopropyl-, Di-n-butyl-, Di-sec.-butyl-, Di-tert.-butyl-, Di-tert.pentyl- und Bis(2-ethylhexyl)dicarbonat, Dicycloalkyldicarbonate, vor allem Di-(C₅-C₈-cycloalkyl)dicarbonate, wie Dicyclopentyl-, Dicyclohexyl- und Dicycloheptyldicarbonat, Dicycloalkylalkyldicarbonate, wie Bis(1- und 2-cyclohexylethyl)- und Bis(1,- 2- und 3-cyclohexylpropyl)dicarbonat, Diaralkyldicarbonate, vor allem Diphenyl-(C₁-C₄-alkyl)dicarbonate, wie Dibenzyl-, Bis(1- und 2-phenylethyl)- und Bis(1-, 2- und 3-phenylpropyl)dicarbonat, und Diphenyldicycloalkyl-(C₁-C₄-alkyl)dicarbonate, wie Bis(1- und 2-cyclohexyl-2-phenyl)-, Bis(1-, 2- und 3-cyclohexyl-2-phenyl)-und Bis(1-, 2- und 3-cyclohexyl-3-phenyl)dicarbonat.

In der Regel werden 2 bis 5 mol, vorzugsweise 3 bis 4 mol, V je mol IV eingesetzt.

Als polares, aprotisches Lösungsmittel eignen sich insbesondere die für Schritt b) genannten Ether, die zweckmäßigerweise in wasserfreier (getrockneter) Form zur Anwendung kommen.

Die Lösungsmittelmenge beträgt üblicherweise 80 bis 300 kg, vorzugsweise 120 bis 200 kg, je kg IV.

Als Base sind vor allem Stickstoffbasen, insbesondere tertiäre aliphatische Amine, bevorzugt Tri-(C₁-C₄-alkyl)amine geeignet, deren Alkylreste gleich oder verschieden sein können und die vorzugsweise in Kombination mit dialkylaminosubstituierten Pyridinen verwendet werden. Ganz besonders bevorzugt werden Kombinationen von Tri-(C₂-C₄-alkyl)aminen, wie Triethyl-, Diisopropylethyl- und Tributylamin, mit 4-(N,N-Dimethylamino)pyridin im Molverhältnis von 4 : 1 bis 1 : 1, insbesondere von etwa 2 : 1, eingesetzt.

Im allgemeinen kommen 5 bis 20 mol-%, vorzugsweise etwa 10 mol-%, Base, bezogen auf V, zum Einsatz.

Die Reaktionstemperatur liegt in der Regel bei 20 bis 70°C, bevorzugt bei 35 bis 50°C.

Die Reaktionszeit beträgt üblicherweise 4 bis 20 h.

Verfahrenstechnisch geht man in Schritt c) zweckmäßigerweise wie folgt vor:

Man legt Lösungsmittel, das Aminorylen-3,4-dicarbonsäureimid IV und Base in einer Schutzgasatmosphäre vor, gibt das Dicarbonat V zu und rührt die Mischung 4 bis 20 h unter Schutzgas bei der gewünschten Reaktionstemperatur. Zur Aufarbeitung auf den Rylenfarbstoff I destilliert man anschließend etwa 70 bis 80 Vol.-% des Lösungsmittels im Vakuum ab, gibt langsam die 2 bis 4-fache Menge eines aliphatischen Alkohols, z.B. Methanol, zu und vervollständigt die Ausfällung des Rylenfarbstoffs I durch Kühlung auf 3 bis 6°C, filtriert den Farbstoff I ab und trocknet bei 100°C im Vakuum.

Die Reinheit der erhaltenen Rylenfarbstoffe I beträgt in der Regel > 97% und ist im allgemeinen für die Anwendung ausreichend. Für besondere Anforderungen ist eine Erhöhung der Reinheit durch Rekristallisation aus einem Halogenkohlenwasserstoff, wie Methylenchlorid und Chloroform, oder einem aromatischen Lösungsmittel, wie Benzol, Toluol und Xylol, oder durch Säulenchromatographie an Kieselgel mit Chloroform als Eluens möglich.

Das erfindungsgemäße Verfahren ermöglicht die Herstellung der Rylenfarbstoffe I und ihrer Zwischenprodukte auf vorteilhafte, wirtschaftliche Weise. Der Wertgehalt der in den einzelnen Verfahrensschritten erhaltenen Produkte liegt ohne weitere Reinigung in der Regel bei > 95%, die Ausbeute über alle Verfahrensschritte, jeweils bezogen auf das eingesetzte Rylen-3,4-dicarbonsäureimidderivat, beträgt im allgemeinen > 60%.

Die erfindungsgemäßen Rylenfarbstoffe I eignen sich hervorragend zur homogenen Einfärbung von hochmolekularen organischen und anorganischen Materialien, insbesondere z.B. von Kunststoffen, vor allem thermoplastischen Kunststoffen, Lacken und Druckfarben sowie oxidischen Schichtsystemen.

Eine besonders vorteilhafte Eigenschaft der erfindungsgemäßen Rylenfarbstoffe I ist ihre Thermochromie, d.h. die irreversible Umwandlung der Farbstoffe von einem molekularen Individuum mit einer Primärfarbe A in ein strukturell unterschiedliches Individuum mit einer Sekundärfarbe B. Der thermochrome Effekt wird durch Erwärmen des eingefärbten Materials auf Temperaturen oberhalb der Umwandlungstemperatur des Rylenfarbstoffs I induziert. Die Primär- und/oder Sekundärfarbe des eingefärbten Materials kann zusätzlich in einfacher Weise variiert werden, indem die erfindungsgemäßen Rylenfarbstoffe I in Mischung untereinander, mit den aus der nicht vorveröffentlichten WO-A-01/16109 bekannten thermochromen Rylenfarbstoffen und/oder mit herkömmlichen Pigmenten und Farbstoffen eingesetzt werden.

Die Thermochromie der erfindungsgemäßen Rylenfarbstoffe I kann außerdem vorteilhaft zur Herstellung lasermarkierbarer bzw. -beschriftbarer Einfärbungen genutzt werden. Durch geschickte Wahl des Substituenten R' kann die Umwandlungstemperatur der Rylenfarbstoffe I für diesen Anwendungszweck gezielt eingestellt werden, was nicht zu erwarten war. So liegen die Umwandlungstemperaturen von erfindungsgemäßen Rylenfarbstoffen der Formel I, in der R' primäres oder sekundäres Alkyl oder Aralkyl bedeutet, im allgemeinen bei > 280°C. Diese Rylenfarbstoffe I können ohne Veränderung der Primärfarbe auf herkömmliche Weise (z.B. durch Extrusion oder Spritzguß) in die klassischen thermoplastischen Kunststoffe (z.B. Polystyrol, Poly(acrylonitril-butadien-styrol), Poly(styrol-acrylonitril), Polycarbonat, Polymethylmethacrylat, Polyethylenterephthalat) eingearbeitet und zur technischen Lasermarkierung bzw. -beschriftung verwendet werden.

Zur Herstellung einer lasermarkierbaren bzw. -beschriftbaren Einfärbung werden die erfindungsgemäßen Rylenfarbstoffe I (bzw. ihre Mischungen untereinander, mit den aus der nicht vorveröffentlichten WO-A-01/16109 her bekannten thermochromen Rylenfarbstoffen und/oder mit anderen Farbmitteln) in Kombination mit einem oder mehreren transparenten oder transluzenten, organischen oder anorganischen (N)IR-Absorbern mit insbesondere neutraler oder im Sichtbaren nur schwach ausgebildeter Eigenfarbe eingesetzt, welche die eingestrahlte (N)IR-Laserenergie in die für die thermochrome Umwandlung notwendige thermische Energie umwandeln.

Hierfür können gängige, kommerziell erhältliche (N)IR-Absorber, z.B. solche aus den Klassen der Methine, Azamethine, Übergangsmetall-Dithiolene, Quadratsäurederivate, Phthalocyanine, Naphthalocyanine, Amidinium- und Iminiumsalze sowie insbesondere Quaterrylentetracarbonsäurederivate verwendet werden. Für einen Einsatz zusammen mit Halbleiterlasern sind dabei Absorber mit einem Absorptionsmaximum bei 780 bis 850 nm und für einen Einsatz zusammen mit gängigen Nd-YAG-Lasern Absorber mit einem Absorptionsmaximum bei etwa 1064 nm, die jeweils eine Gramm-Absorptivität am Absorptionsmaximum von mindestens 50 aufweisen, besonders bevorzugt.

### Beispiele

### A) Herstellung von erfindungsgemäßen Rylenfarbstoffen der Formel I

### a) Herstellung der Ketimine

### Beispiele 1 bis 6

Eine unter Schutzgas gerührte Lösung von k mmol des Übergangsmetallkatalysators Tris(benzylidenaceton)dipalladium(0) und c µmol des Cokatalysators 2,2''-Bis(diphenylphosphino)-1,1'-binaphthyl (Racemat) in a₁ 1 wasserfreiem Toluol wurde nach Zugabe von x₁ g (18 mmol) des Monobromrylen-3,4-dicarbonsäureimids II, 6,52 g (36 mmol) Benzophenonimin und 3,46 g Natrium-tert.-butanolat 12 h auf 100°C erhitzt.

Nach Abkühlen auf Raumtemperatur, Abfiltrieren der unlöslichen Bestandteile und Abdestillieren des Lösungsmittels unter vermindertem Druck wurde das Rohprodukt in möglichst wenig Chloroform oder Methylenchlorid unter leichtem Erwärmen gelöst. Nach Filtration wurde das Produkt durch vorsichtigen Zusatz der fünffachen Menge an Petrolether (Siedebereich 60-90°C) wieder ausgefällt, abfiltriert und bei 100°C im vakuum getrocknet.

Weitere Einzelheiten zu diesen Versuchen sowie deren Ergebnisse sind in Tabelle 1 zusammengestellt.

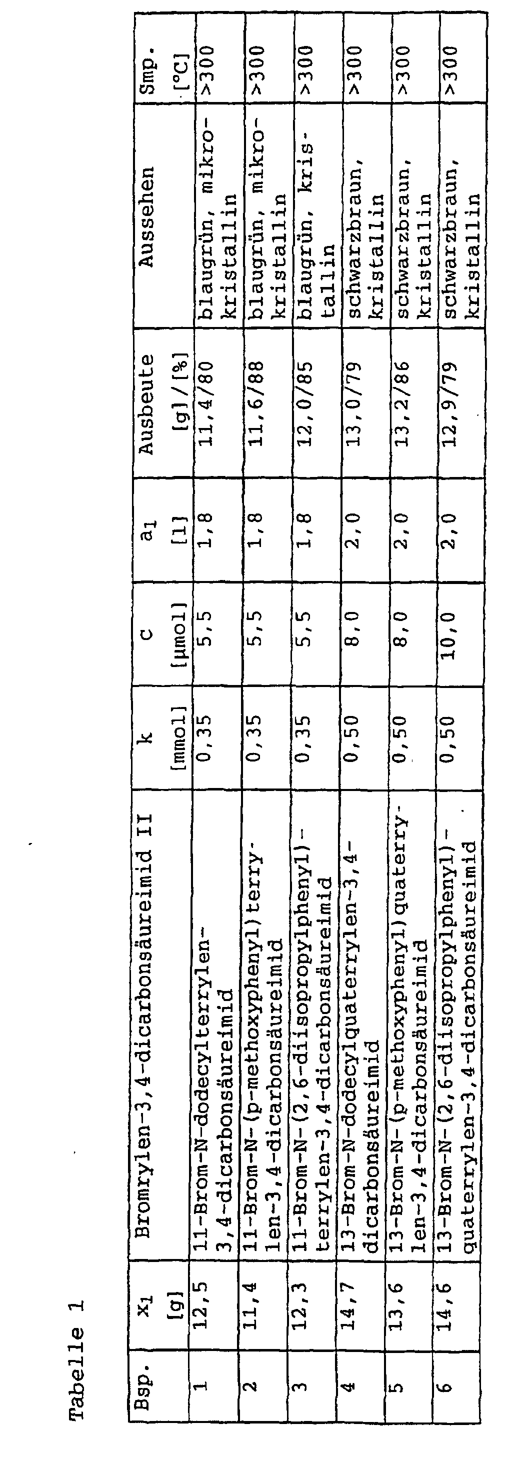

### Analytische Daten zu Beispiel 1:

11-(Diphenylmethylenimino)-N-dodecylterrylen-3,4-dicarbonsäureimid:
Elementaranalyse (Gew.-% ber./gef.):
C: 86,3/86,1; H: 6,1/6,2; N: 3,5/3,5; O: 4,0/4,1;
Masse (FD, 8kV): m/z = 792,4 (M⁺, 100 %).

### Analytische Daten zu Beispiel 2:

11-(Diphenylmethylenimino)-N-(p-methoxyphenyl)terrylen-3,4-dicarbonsäureimid:
Elementaranalyse (Gew.-% ber./gef.):
C: 85,5/85,4; H: 4,1/4,0; N: 3,8/3,9; O: 6,6/6,7;
Masse (FD, 8kV): m/z = 730,4 (M⁺, 100 %).

### Analytische Daten zu Beispiel 3:

11-(Diphenylmethylenimino)-N-(2,6-diisopropylphenyl)terrylen-3,4-dicarbonsäureimid:
Elementaranalyse (Gew.-% ber./gef.):
C: 87,2/87,0; H: 5,1/5,2; N: 3,6/3,6; O: 4,1/4,2;
Masse (FD, 8kV): m/z = 784,3 (M⁺, 100 %).

### Analytische Daten zu Beispiel 4:

13-(Diphenylmethylenimino)-N-dodecylquaterrylen-3,4-dicarbonsäureimid:
Elementaranalyse (Gew.-% ber./gef.):
C: 87,7/87,4; H: 5,7/5,8; N: 3,0/3,1; O: 3,5/3,7;
Masse (FD, 8kV): m/z = 916,4 (M⁺, 100 %).

### Analytische Daten zu Beispiel 5:

13-(Diphenylmethylenimino)-N-(p-methoxyphenyl)quaterrylen-3,4-dicarbonsäureimid:
Elementaranalyse (Gew.-% ber./gef.):
C: 87,1/87,3; H: 4,0/3,9; N: 3,3/3,3; O: 5,6/5,5;
Masse (FD, 8kV): m/z = 854,2 (M⁺, 100 %).

### Analytische Daten zu Beispiel 6:

13-(Diphenylmethylenimino)-N-(2,6-diisopropylphenyl)quaterrylen-3,4-dicarbonsäureimid:
Elementaranalyse (Gew.-% ber./gef.):
C: 88,5/88,0; H: 4,9/5,0; N: 3,1/3,0; O: 3,5/3,9;
Masse (FD, 8kV): m/z = 908,3 (M⁺, 100 %).

### b) Herstellung der Aminorylen-3,4-dicarbonsäureimide IV

### Beispiele 7 bis 12

Eine Lösung von 10 g (x₂ mmol) des Ketimins aus Beispiel 1 bis 6 in a₂ 1 1,4-Dioxan wurde nach Zugabe von 50 ml 2-molarer wäßriger Salzsäure t₂ h bei T₂°C gerührt.

Nach Neutralisation des Reaktionsgemischs mit 25 gew.-%iger wäßriger Ammoniaklösung und Abdestillieren des Lösungsmittels im Vakuum wurde der Rückstand zur Entfernung von anorganischen Verunreinigungen in einer Mischung aus 1 1 Wasser und 50 ml konzentrierter wäßriger Ammoniaklösung suspendiert, abfiltriert, erneut zweimal mit zwischengeschalteter Filtration in jeweils 1 1 heißer 20 gew.-%iger wäßriger Ammoniaklösung suspendiert und dann filtriert. Durch Heißextraktion mit Petrolether (Siedebereich 60-90°C) wurde das Rohprodukt anschließend von Benzophenon und anderen organischen Verunreinigungen befreit und dann bei 100°C im Vakuum getrocknet.

Weitere Einzelheiten zu diesen Versuchen sowie deren Ergebnisse sind in Tabelle 2 zusammengestellt.

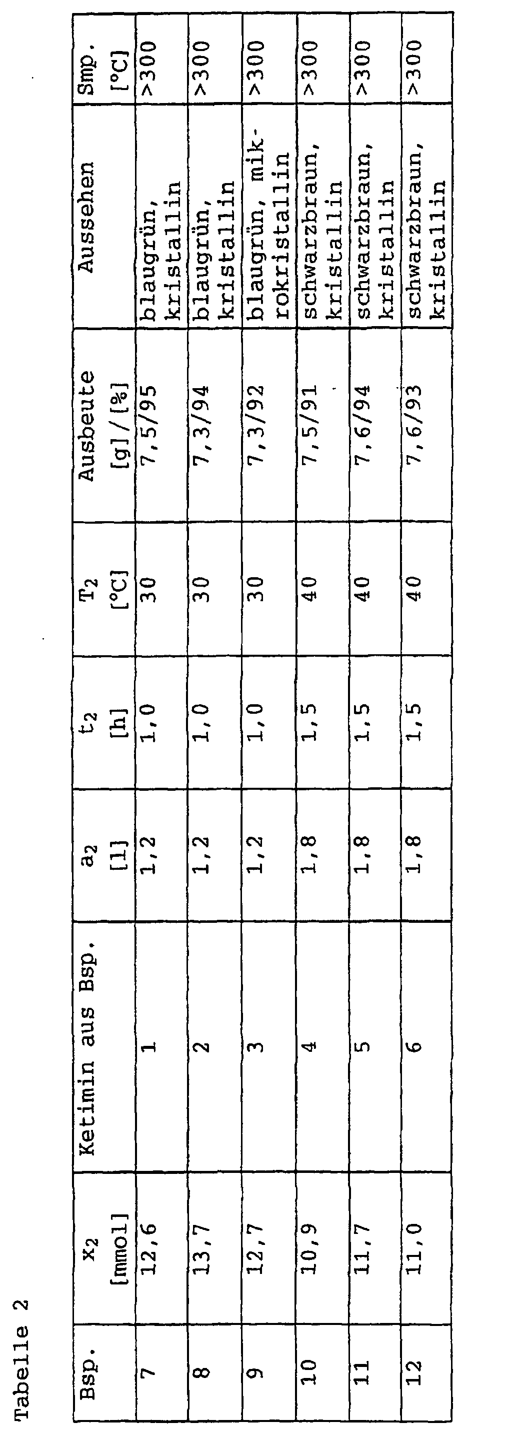

### Analytische Daten zu Beispiel 7:

11-Amino-N-dodecylterrylen-3,4-dicarbonsäureimid:
Elementaranalyse (Gew.-% ber./gef.):
C: 84,0/84,2; H: 6,4/6,5; N: 4,5/4,3; O: 5,1/4,9;
Masse (FD, 8kV) : m/z = 628,4 (M⁺, 100 %).

### Analytische Daten zu Beispiel 8:

11-Amino-N-(p-methoxyphenyl)terrylen-3,4-dicarbonsäureimid:
Elementaranalyse (Gew.-% ber./gef.):
C: 82,7/82,3; H: 3,9/4,1; N: 4,9/5,1; O: 8,5/8,4;
Masse (FD, 8kV): m/z = 566,2 (M⁺, 100 %).

### Analytische Daten zu Beispiel 9:

11-Amino-N-(2,6-diisopropylphenyl)terrylen-3,4-dicarbonsäureimid:
Elementaranalyse (Gew.-% ber./gef.):
C: 87,2/87,0; H: 5,1/5,2; N: 3,6/3,6; 0: 4,1/4,2;
Masse (FD, 8kV): m/z = 620,3 (M⁺, 100 %).

### Analytische Daten zu Beispiel 10:

13-Amino-N-dodecylquaterrylen-3,4-dicarbonsäureimid:
Elementaranalyse (Gew.-% ber./gef.):
C: 86,1/86,4; H: 5,9/5,7; N: 3,7/3,7; O: 4,3/4,2;
Masse (FD, 8kV): m/z = 752,6 (M⁺, 100 %).

### Analytische Daten zu Beispiel 11:

13-Amino-N-(p-methoxyphenyl)quaterrylen-3,4-dicarbonsäureimid:
Elementaranalyse (Gew.-% ber./gef.):
C: 85,2/85,2; H: 3,8/3,9; N: 4,1/4,0; O: 7,0/6,9;
Masse (FD, 8kV) : m/z = 690,3 (M⁺, 100 %).

### Analytische Daten zu Beispiel 12:

13-Amino-N-(2,6-diisopropylphenyl)quaterrylen-3,4-dicarbonsäureimid:
Elementaranalyse (Gew.-% ber./gef.):
C: 88,5/88,0; H: 4,9/5,0; N: 3,1/3,0; O: 3,5/3,9;
Masse (FD, 8kV) : m/z = 744,4 (M⁺, 100 %).

### c) Herstellung der Rylenfarbstoffe I

### Beispiele 13 bis 25

Eine unter Schutzgas gerührte Lösung von 49 mg (0,4 mmol) 4-(N,N-Dimethylamino)pyridin, 408 mg (0,8 mmol) Triethylamin und x₃ g (2 mmol) des Aminorylen-3,4-dicarbonsäureimids aus Beispiel 7 bis 12 in 200 ml wasserfreiem Dioxan wurde nach Zugabe von y g (8 mmol) des Dicarbonats V t₃ h auf 45°C erhitzt.

Nach Abdestillieren von 80 Vol.-% des Lösungsmittels unter Vakuum wurde die Ausfällung des Produkts durch langsamen Zusatz von 500 ml Methanol und Kühlung auf 3 bis 6°C vervollständigt. Das ausgefallene Produkt wurde abfiltriert, mit Methanol gewaschen und bei 100°C im Vakuum getrocknet.

Die Schmelzpunkte aller erhaltenen Rylenfarbstoffe I lagen oberhalb der Temperatur der thermischen Umwandlung (Abspaltung von CO₂ und Alken bzw. Aralken).

Weitere Einzelheiten zu diesen Versuchen sowie deren Ergebnisse sind in Tabelle 3 zusammengestellt.

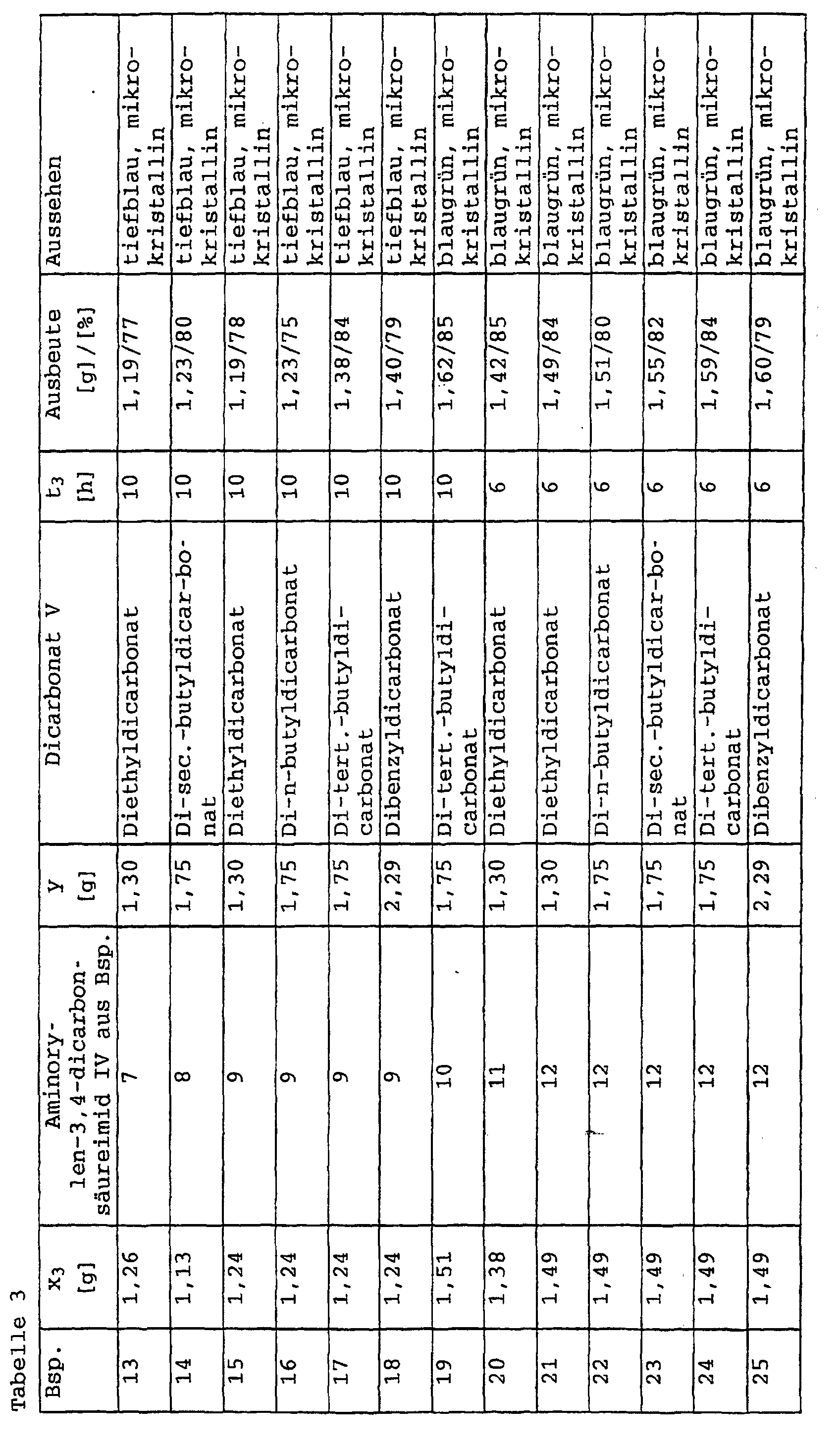

### Analytische Daten zu Beispiel 13:

11-(Diethoxycarbonyl)amino-N-(dodecyl)terrylen-3,4-dicarbonsäureimid:
Elementaranalyse (Gew.-% ber./gef.):
C: 77,7/77,7; H: 6,3/6,5; N: 3,6/3,5; O: 12,4/12,2;
Masse (MALDI-TOF): m/z = 772,2 (M⁺, 100 %);
IR (KBr): ν = 1698 (s, C=O), 1665 (s, C=O), 1500 (s) cm⁻¹;
UV/VIS (NMP): λₘₐₓ (ε) = 580 (44000), 646 (49900) nm.

### Analytische Daten zu Beispiel 14:

11-(Di-sec.-butoxycarbonyl)amino-N-(4-methoxyphenyl)terrylen-3,4-dicarbonsäureiimid:
Elementaranalyse (Gew.-% ber./gef.):
C: 76,8/76,7; H: 5,0/4,9; N: 3,7/3,7; O: 14,6/14,7;
Masse (MALDI-TOF): m/z = 766,6 (M⁺, 100 %);
IR (KBr): ν = 1701 (s, C=O), 1668 (s, C=O) cm⁻¹;
UV/VIS (NMP): λₘₐₓ (ε) = 576 (44370), 639 (43530) nm.

### Analytische Daten zu Beispiel 15:

11-(Diethoxycarbonyl)amino-N-(2,6-diisopropylphenyl)terrylen-3,4-dicarbonsäureimid:
Elementaranalyse (Gew.-% ber./gef.):
C: 78,5/78,3; H: 5,3/5,3; N: 3,65/3,7; 0: 12,55/12,7;
Masse (MALDI-TOF): m/z = 764,3 (M⁺, 100 %);
IR (KBr): ν = 1700 (s, c=O), 1666 (s, C=O), 1501 (s) cm⁻¹;
UV/VIS (NMP): λₘₐₓ (ε) = 577 (45080), 644 (46110) nm.

### Analytische Daten zu Beispiel 16:

11-(Di-n-butoxycarbonyl)amino-N-(2,6-diisopropylphenyl)terrylen-3,4-dicarbonsäureimid:
Elementaranalyse (Gew.-% ber./gef.):
C: 79,0/79,3; H: 5,9/5,8; N: 3,4/3,5; O: 11,7/11,4;
Masse (MALDI-TOF): m/z = 820,1 (M⁺, 100 %);
IR (KBr): ν = 1702 (s, C=O), 1667 (s, C=O), 1499 (s) cm⁻¹;
UV/VIS (NMP): λₘₐₓ (ε) = 577 (43980), 645 (44230) nm.

### Analytische Daten zu Beispiel 17:

11-(Di-tert.-butoxycarbonyl)amino-N-(2,6-diisopropylphenyl)terrylen-3,4-dicarbonsäureimid:
Elementaranalyse (Gew.-% ber./gef.):
C: 79,0/78,8; H: 5,9/6,0; N: 3,4/3,5; O: 11,7/11,7;
Masse (MALDI-TOF): m/z = 820,5 (M⁺, 100 %);
IR (KBr): ν = 1749 (s, C=O), 1702 (s, C=O), 1664 (s, C=O), 1594 (s, C=O) cm⁻¹;
UV/VIS (NMP): λₘₐₓ (ε) = 579 (44270), 643 (44120) nm.

### Analytische Daten zu Beispiel 18:

11-(Dibenzyloxycarbonyl)amino-N-(2,6-diisopropylphenyl)terrylen-3,4-dicarbonsäureimid:
Elementaranalyse (Gew.-% ber./gef.):
C: 81,1/80,7; H: 5,0/5,1; N: 3,2/3,2; O: 10,8/11,0;
Masse (MALDI-TOF): m/z = 888,6 (M⁺, 100 %);
IR (KBr): ν = 1700 (s, C=O), 1665 (s, C=O), 1498 (s) cm⁻¹;
UV/VIS (NMP): λₘₐₓ (ε) = 577 (43980), 645 (46800) nm.

### Analytische Daten zu Beispiel 19:

13-(Di-tert.-butoxycarbonyl)amino-N-(dodecyl)quaterrylen-3,4-dicarbonsäureimid:
Elementaranalyse (Gew.-% ber./gef.):
C: 80,7/81,0; H: 6,3/6,1; N: 2,9/2,8; O: 10,1/10,1;
Masse (MALDI-TOF): m/z = 952,2 (M⁺, 100 %);
IR (KBr): ν = 1748 (s, C=O), 1699 (s, C=O), 1665 (s, C=O), 1593 (s, C=O) cm⁻¹;
UV/VIS (NMP): λₘₐₓ (ε) = 680 (75000), 744 (78760) nm.

### Analytische Daten zu Beispiel 20:

13-(Diethoxycarbonyl)amino-N-(4-methoxyphenyl)quaterrylen-3,4-dicarbonsäureimid:
Elementaranalyse (Gew.-% ber./gef.):
C: 79,1/78,7; H: 4,1/4,3; N: 3,4/3,5; O: 13,4/13,4;
Masse (MALDI-TOF): m/z = 834,4 (M⁺, 100 %);
IR (KBr): ν = 1700 (s, C=O), 1667 (s, C=O), 1504 (s) cm⁻¹;
UV/VIS (NMP): λₘₐₓ (ε) = 677 (75580), 738 (73920) nm.

### Analytische Daten zu Beispiel 21:

13-(Diethoxycarbonyl)amino-N-(2,6-diisopropylphenyl)quaterrylen-3,4-dicarbonsäureimid:
Elementaranalyse (Gew.-% ber./gef.):
C: 81,05/81,2; H: 5,0/5,0; N: 3,15/3,1; O: 10,8/10,7;
Masse (FD, 8kV): m/z = 888,4 (M⁺, 100 %);
IR (KBr): ν = 1701 (s, C=O), 1667 (s, C=O), 1502 (s) cm⁻¹;
UV/VIS (NMP): λₘₐₓ (ε) = 678 (74080), 742 (77640) nm.

### Analytische Daten zu Beispiel 22:

13-(Di-n-butoxycarbonyl)amino-N-(2,6-diisopropylphenyl)quaterrylen-3,4-dicarbonsäureimid:
Elementaranalyse (Gew.-% ber./gef.):
C: 81,3/81,0; H: 5,5/6,0; N: 3,0/3,0; O: 10,2/10,0;
Masse (FD, 8kV) : m/z = 944,3 (M⁺, 100 %);
IR (KBr): ν = 1701 (s, C=O), 1668 (s, C=O), 1504 (s) cm⁻¹;
UV/VIS (NMP): λₘₐₓ (ε) = 678 (72840), 741 (75210) nm.

### Analytische Daten zu Beispiel 23:

13-(Di-sec.-butoxycarbonyl)amino-N-(2,6-diisopropylphenyl)-quaterrylen-3,4-dicarbonsäureimid:
Elementaranalyse (Gew.-% ber./gef.):
C: 81,3/81,1; H: 5,6/5,4; N: 3,0/3,1; O: 10,1/10,3;
Masse (FD, 8kV): m/z = 944,2 (M⁺, 100 %);
IR (KBr): ν = 1702 (s, C=O), 1666 (s, C=O) cm⁻¹;
UV/VIS (NMP): λₘₐₓ (ε) = 679 (73050), 740 (74990) nm.

### Analytische Daten zu Beispiel 24:

13-(Di-tert.-butoxycarbonyl)amino-N-(2,6-diisopropylphenyl)-quaterrylen-3,4-dicarbonsäureimid:
Elementaranalyse (Gew.-% ber./gef.):
C: 81,3/81,0; H: 5,6/5,9; N: 3,0/3,0; O: 10,1/10,1;
Masse (FD, 8kV): m/z = 944,4 (M⁺, 100 %);
IR (KBr): ν = 1750 (s, C=O), 1700 (s, C=O), 1665 (s, C=O), 1593 (s, C=O) cm⁻¹;
UV/VIS (NMP): λₘₐₓ (ε) = 680 (73820), 745 (73670) nm.

### Analytische Daten zu Beispiel 25:

13-(Di-benzyloxycarbonyl)amino-N-(2,6-diisopropylphenyl)-quaterrylen-3,4-dicarbonsäureimid:
Elementaranalyse (Gew.-% ber./gef.):
C: 83,0/83,3; H: 4,8/5,0; N: 2,8/2,7; O: 9,5/9,0;
Masse (FD, 8kV): m/z = 1012,7 (M⁺, 100 %);
IR (KBr): ν = 1699 (s, C=O), 1666 (s, C=O), 1501 (s) cm⁻¹;
UV/VIS (NMP): λₘₐₓ (ε) = 678 (73550), 742 (75800) nm.

### B) Anwendung von erfindungsgemäßen Rylenfarbstoffen I

### a) Herstellung von thermochrom eingefärbten hochmolekularen Materialien

Zur Herstellung thermochrom eingefärbter thermoplastischer Kunststoffe wurden jeweils x g des Farbstoffs I sowie gegebenenfalls z g des transparenten Pigments P oder eines der thermochromen Farbstoffe F aus der nicht vorveröffentlichten WO-A-01/16109 mit 100 g eines der Matrixpolymeren
- PS:: Polystyrol 144C glasklar (BASF)
- PMMA:: Polymethylmethacrylat Formmasse 7N glasklar (Röhm) bzw.
- PC:: Polycarbonat Makrolon® 2858 (Bayer)
vermischt und auf konventionelle Weise durch Extrusion und Spritzguß in ein Halbzeug überführt.

Zur Herstellung thermochromer Lackierungen wurde eine Mischung von jeweils x g des Farbstoffs I und 100 g eines Alkyd-Melamin-Einbrennlacks auf Lösungsmittelbasis (45 Gew.-% Feststoffanteil) mit 150 g Glaskugeln (3 mm Durchmesser) 30 min mit einem Skandexgerät geschüttelt, dann mit einer Rakel auf Blech übertragen und 30 min bei 130°C eingebrannt (Schichtdicke im getrockneten Zustand 55 ± 5 µm).

Die thermochrome Farbänderung (Primärfarbe → Sekundärfarbe) der eingefärbten polymeren Systeme wurde durch 15-minütiges Tempern auf die jeweilige Umwandlungstemperatur T°C induziert.

Weitere Einzelheiten zu diesen Versuchen sowie deren Ergebnisse sind in Tabelle 4 zusammengestellt.

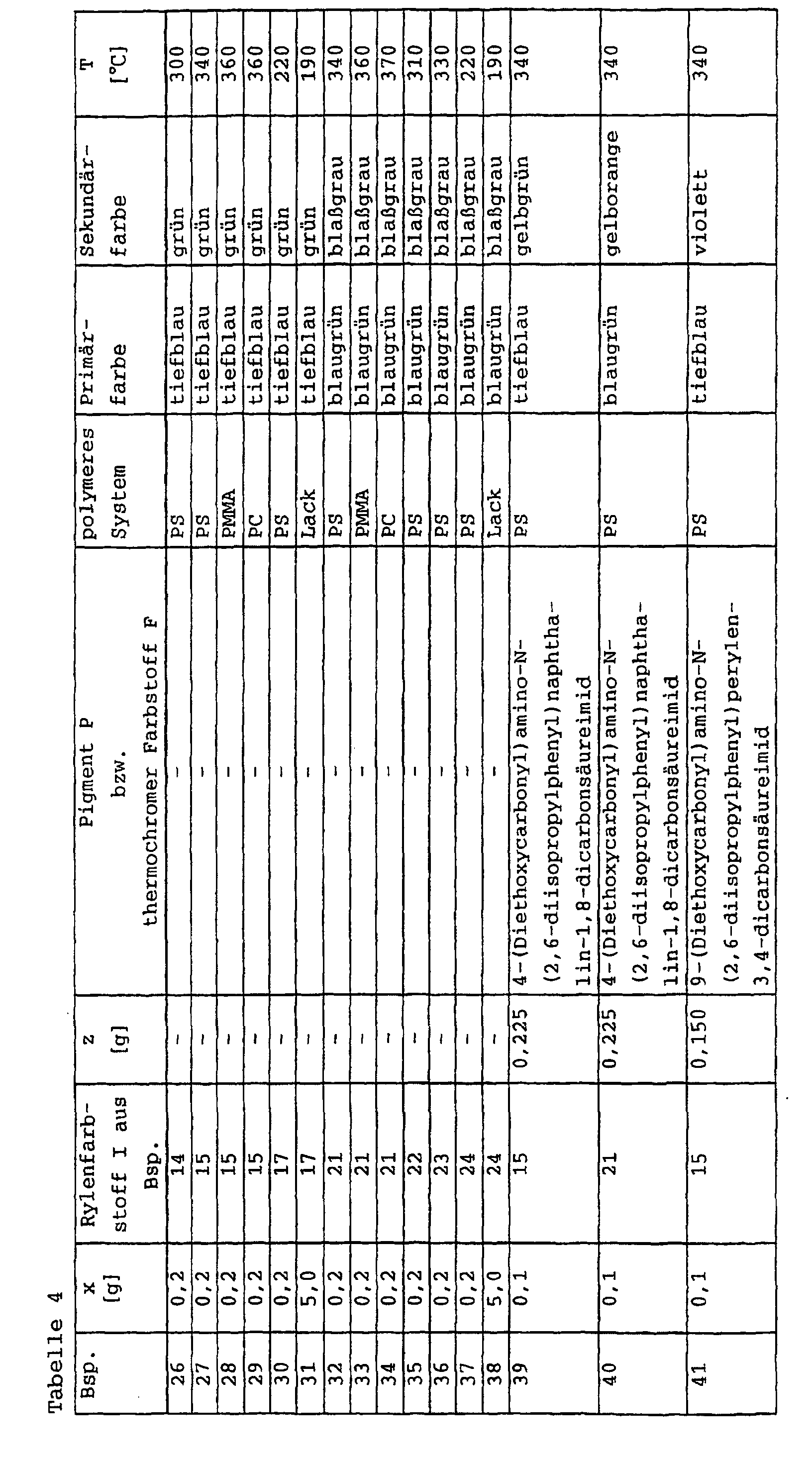

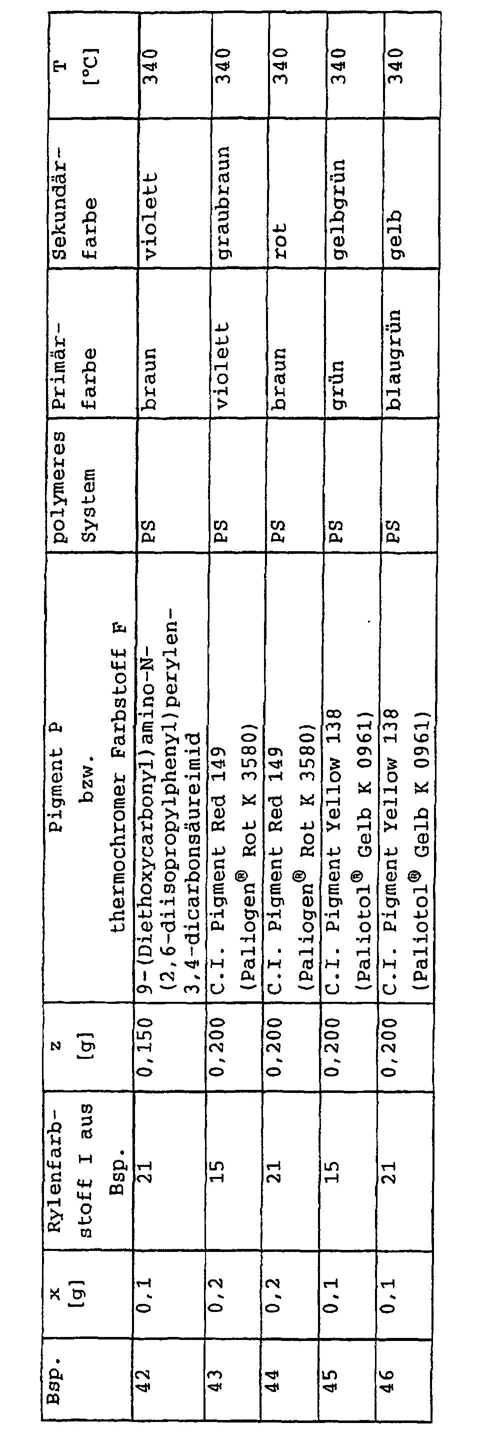

### b) Herstellung von lasermarkierbaren bzw. -beschriftbaren Einfärbungen

Zur Herstellung lasermarkierbarer bzw. -beschriftbarer Einfärbungen wurden die Farbstoffe bzw. Farbstoffmischungen aus Beispiel 28, 33, 39 bzw. 41, wie unter a) beschrieben, jedoch unter Zusatz von y g des (Nah) Infrarot-Absorbers A in PMMA (Beispiele 28 und 33) bzw. PS (Beispiele 39 und 41) eingearbeitet.

Anschließend wurde das eingefärbte Halbzeug mit einem Nd-YAG-Laser (Emissionswellenlänge von 1064 nm, Lasernennleistung 40 Watt; Scanrate 1000 mm/s; Beispiele 47 bis 50) bzw. mit einer Halbleiterlaserdiode (Emissionswellenlänge von 780 nm, Lasernennleistung 1 Watt, Scanrate 100 mm/s; Beispiele 51 bis 54) markiert.

Weitere Einzelheiten zu diesen Versuchen sowie deren Ergebnisse sind in Tabelle 5 zusammengestellt.

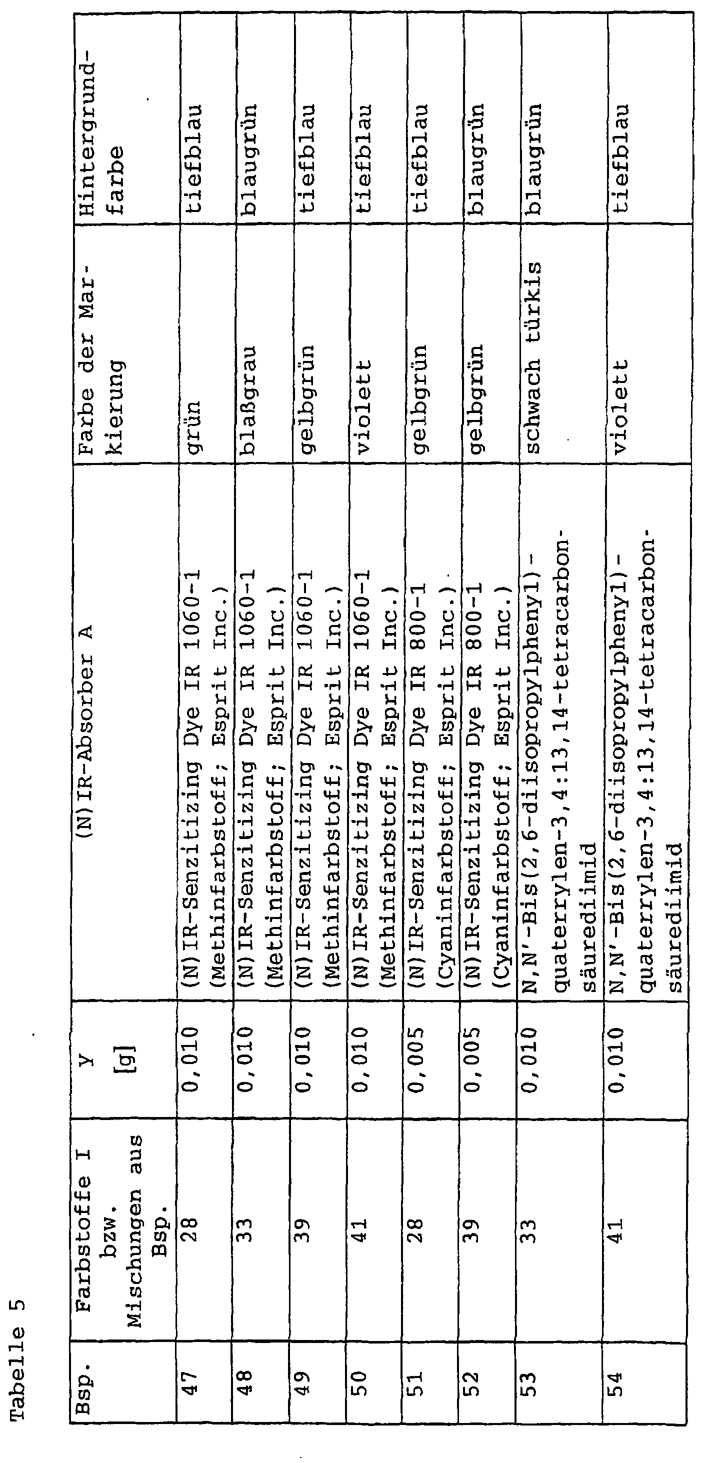

## Patentansprüche

1. Rvlenfarbstoffe der allgemeinen Formel I 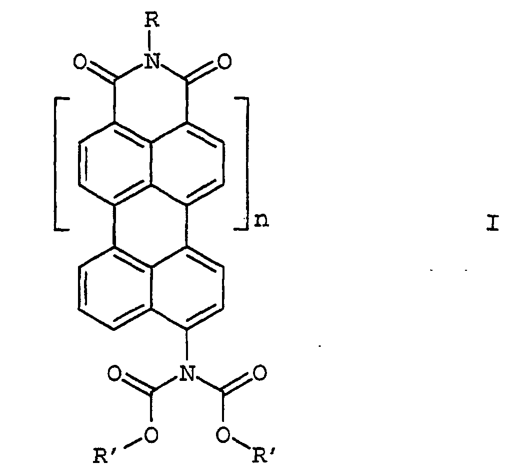 in der die Variablen folgende Bedeutung haben:
R Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
Aryl oder Hetaryl, das jeweils durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy und/oder Cyano substituiert sein kann, ein- oder mehrfach substituiert sein kann;
R' C₂-C₃₀-Alkyl, dessen Kohlenstoffkette durch eine oder mehrere Gruppierungen -O- und/oder -CO- unterbrochen sein kann und das durch Cyano, C₁-C₆-Alkoxy, C₅-C₈-Cycloalkyl, dessen Kohlenstoffgerüst durch eine oder mehrere Gruppierungen -O-, -S- und/oder -NR¹- unterbrochen sein kann und das durch C₁-C₆-Alkyl substituiert sein kann, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
Methyl, das durch Aryl, Hetaryl und/oder C₅-C₈-Cycloalkyl, das jeweils durch C₁-C₁₈-Alkyl oder C₁-C₆-Alkoxy substituiert sein kann, ein- oder zweifach substituiert ist;
C₅-C₈-Cycloalkyl, dessen Kohlenstoffgerüst durch eine oder mehrere Gruppierungen -O-, -S- und/oder -NR¹- unterbrochen sein kann und das durch C₁-C₆-Alkyl ein- oder mehrfach substituiert sein kann;
R¹ Wasserstoff oder C₁-C₆-Alkyl;
R² Wasserstoff; C₁-C₁₈-Alkyl; Aryl oder Hetaryl, das jeweils durch C₁-C₆-Alkyl, C₁-C₆-Alkoxy und/oder Cyano substituiert sein kann:
n 2 oder 3.

2. Rylenfarbstoffe der Formel I nach Anspruch 1, in der die Variablen folgende Bedeutung haben:
R Wasserstoff;
C₁-C₃₀-Alkyl, dessen Kohlenstoffkette durch eine oder mehrere Gruppierungen -O- und/oder -CO- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
Aryl oder Hetaryl, das jeweils durch C₁-C₁₈-Alkyl, C₁-C₆-Alkoxy, Cyano, -CONHR² und/oder -NHCOR² ein- oder mehrfach substituiert sein kann;
R' C₂-C₃₀-Alkyl, dessen Kohlenstoffkette durch eine oder mehrere Gruppierungen -O- und/oder -CO- unterbrochen sein kann und das durch C₁-C₆-Alkoxy, C₅-C₈-Cycloalkyl, dessen Kohlenstoffgerüst durch eine oder mehrere Gruppierungen -O- und/oder -NR¹- unterbrochen sein kann und das durch C₁-C₆-Alkyl substituiert sein kann, und/oder Aryl, das durch C₁-C₁₈-Alkyl oder C₁-C₆-Alkoxy substituiert sein kann, ein- oder mehrfach substituiert sein kann;
Methyl, das durch Aryl und/oder C₅-C₈-Cycloalkyl, das jeweils durch C₁-C₁₈-Alkyl oder C₁-C₆-Alkoxy substituiert sein kann, ein- oder zweifach substituiert ist;
C₅-C₈-Cycloalkyl, dessen Kohlenstoffgerüst durch eine oder mehrere Gruppierungen -O- und/oder -NR¹- unterbrochen sein kann und das durch C₁-C₆-Alkyl ein- oder mehrfach substituiert sein kann.

3. Rylenfarbstoffe der Formel I nach Anspruch 1, in der die Variablen folgende Bedeutung haben:
R Wasserstoff;
C₁-C₃₀-Alkyl, dessen Kohlenstoffkette durch eine oder mehrere Gruppierungen -O- und/oder -CO- unterbrochen sein kann und das durch C₁-C₆-Alkoxy und/oder Aryl, das durch C₁-C₁₈-Alkyl oder C₁-C₆-Alkoxy substituiert sein kann, ein- oder zweifach substituiert ist;
Aryl, das durch C₁-C₁₈-Alkyl, C₁-C₆-Alkoxy und/oder Cyano ein- oder mehrfach substituiert sein kann;
R' C₂-C₃₀-Alkyl, dessen Kohlenstoffkette durch eine oder mehrere Gruppierungen -O- unterbrochen sein kann und das durch C₁-C₆-Alkoxy, C₅-C₈-Cycloalkyl, das durch C₁-C₆-Alkyl substituiert sein kann, und/oder Aryl, das durch C₁-C₁₈-Alkyl oder C₁-C₆-Alkoxy substituiert sein kann, ein- oder mehrfach substituiert sein kann;
Methyl, das durch Aryl und/oder C₅-C₈-Cycloalkyl, das jeweils durch C₁-C₁₈-Alkyl oder C₁-C₆-Alkoxy substituiert sein kann, ein- oder zweifach substituiert ist;
C₅-C₈-Cycloalkyl, das durch C₁-C₆-Alkyl ein- oder mehrfach substituiert sein kann.

4. Verfahren zur Herstellung von Rylenfarbstoffen der Formel I gemäß den Ansprüchen 1 bis 3, **dadurch gekennzeichnet, daß** man
a) ein Bromrylen-3,4-dicarbonsäureimid der allgemeinen Formel II 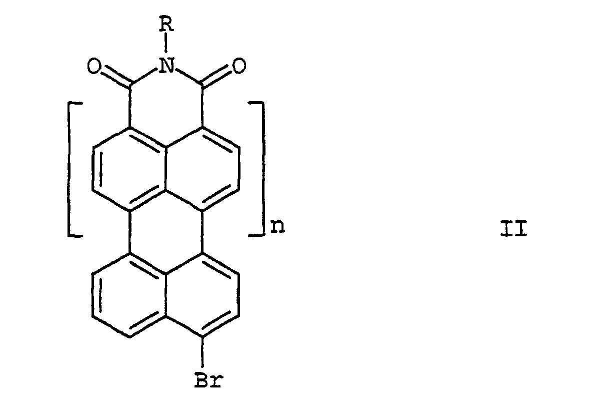 in Gegenwart eines aprotischen organischen Lösungsmittels, eines Übergangsmetallkatalysatorsystems und einer Base in einer Aryl-N-Kupplungsreaktion mit einem Benzophenonimin der allgemeinen Formel III 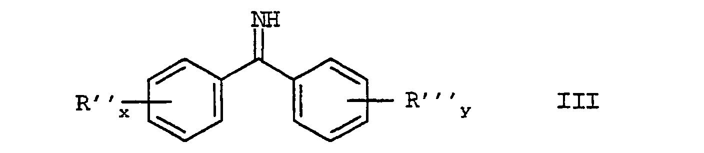 in der die Variablen
R'', R''' unabhängig voneinander Wasserstoff, C₁-C₆-Alkyl oder C₁-C₆-Alkoxy und
x, y unabhängig voneinander eine ganze Zahl von 1 bis 3 bedeuten,
umsetzt,
b) das gebildete Ketimin in Gegenwart einer Säure und eines polaren, aprotischen Lösungsmittels zu einem Aminorylen-3,4-dicarbonsäureimid der allgemeinen Formel IV 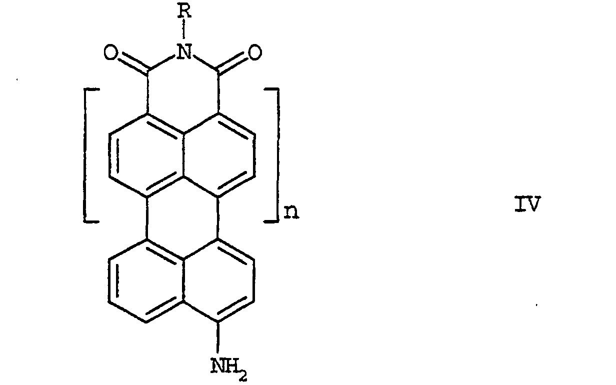 hydrolysiert und
c) dieses anschließend in Gegenwart eines polaren, aprotischen Lösungsmittels und einer Base mit einem Dicarbonat der allgemeinen Formel V 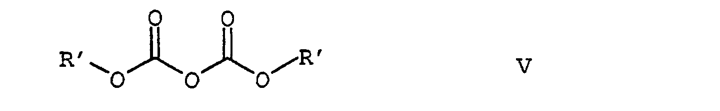 zu dem Rylenfarbstoff I umsetzt.

5. Verfahren zur Herstellung von Aminorylen-3,4-dicarbonsäureimiden der allgemeinen Formel IV 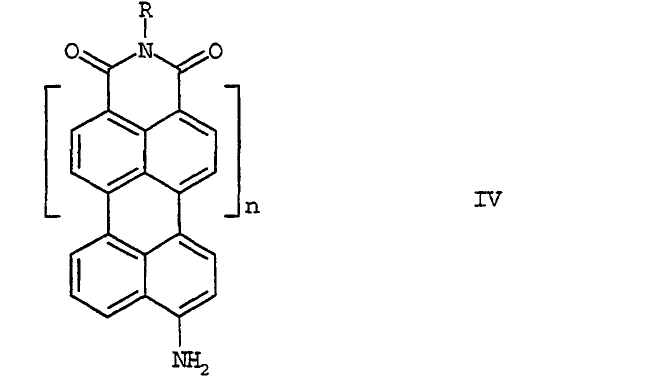 in der die Variablen folgende Bedeutung haben:
R Wasserstoff; C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
Aryl oder Hetaryl, das jeweils durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy und/oder Cyano substituiert sein kann, ein- oder mehrfach substituiert sein kann;
R¹ Wasserstoff oder C₁-C₆-Alkyl;
R² Wasserstoff; C₁-C₁₈-Alkyl; Aryl oder Hetaryl, das jeweils durch C₁-C₆-Alkyl, C₁-C₆-Alkoxy und/oder Cyano substituiert sein kann;
n 2 oder 3,
**dadurch gekennzeichnet, daß** man
a) ein Bromrylen-3,4-dicarbonsäureimid der allgemeinen Formel II 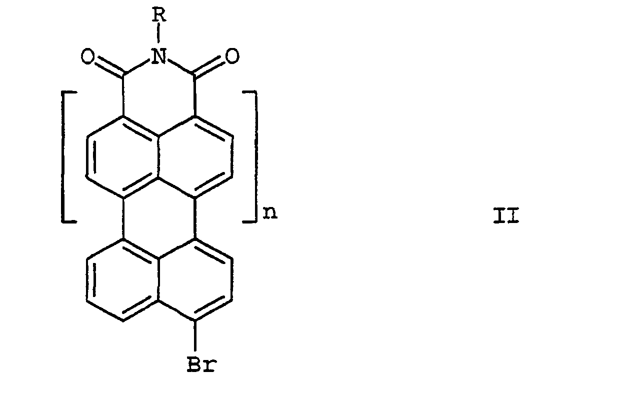 in Gegenwart eines aprotischen organischen Lösungsmittels, eines Übergangsmetallkatalysatorsystems und einer Base in einer Aryl-N-Kupplungsreaktion mit einem Benzophenonimin der allgemeinen Formel III 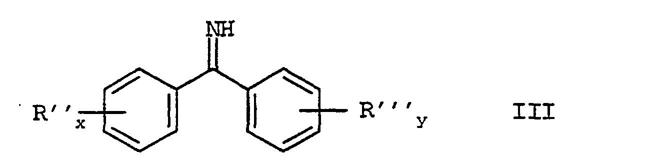 umsetzt und
b) das gebildete Ketimin in Gegenwart einer Säure und eines polaren, aprotischen Lösungsmittels hydrolysiert.

6. Aminorylen-3,4-dicarbonsäureimide der allgemeinen Formel IV 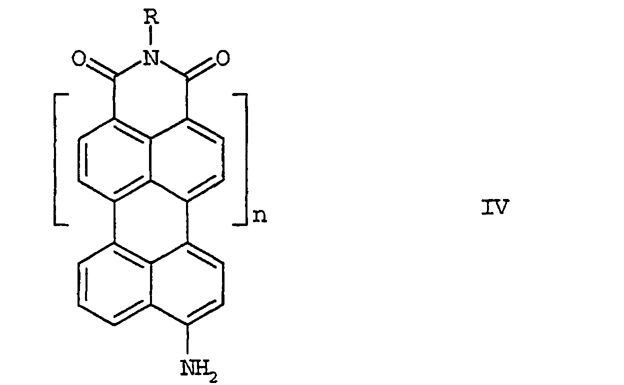 in der die Variablen folgende Bedeutung haben:
R Wasserstoff; C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
Aryl oder Hetaryl, das jeweils durch C₁-C₁₈-Alkyl, C₁-C₆-Alkoxy, Cyano, -CONHR², -NHCOR² und/oder Aryl- oder Hetarylazo, das jeweils durch C₁-C₁₀-Alkyl, C₁-C₆-Alkoxy und/oder Cyano substituiert sein kann, ein- oder mehrfach substituiert sein kann;
R¹ Wasserstoff oder C₁-C₆-Alkyl;
R² Wasserstoff; C₁-C₁₈-Alkyl; Aryl oder Hetaryl, das jeweils durch C₁-C₆-Alkyl, C₁-C₆-Alkoxy und/oder Cyano substituiert sein kann;
n 2 oder 3.

7. Verwendung von Rylenfarbstoffen der Formel I gemäß den Ansprüchen 1 bis 3 zur Einfärbung von hochmolekularen organischen und anorganischen Materialien.

8. Verwendung nach Anspruch 7, **dadurch gekennzeichnet, daß** Kunststoffe, Lacke, Druckfarben und oxidische Schichtsysteme eingefärbt werden.

9. Verwendung nach Anspruch 8 oder 9, **dadurch gekennzeichnet, daß** lasermarkierbare und laserbeschriftbare Einfärbungen hergestellt werden.

## Claims

1. A rylene dye of the general formula I 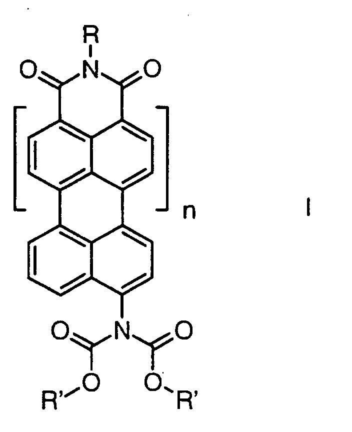 where
R is hydrogen;
C₁-C₃₀-alkyl, whose carbon chain may be interrupted by one or more -O-, -S-, -NR¹-, -CO- and/or -SO₂- groups and which may be monosubstituted or polysubstituted by cyano, C₁-C₆-alkoxy, aryl, which may be substituted by C₁-C₁₈-alkyl or C₁-C₆-alkoxy, and/or a 5- to 7-membered heterocyclic radical which is bonded via a nitrogen atom and may contain further heteroatoms and may be aromatic;
aryl or hetaryl, each of which may be monosubstituted or polysubstituted by C₁-C₁₈-alkyl, C₁-C₆-alkoxy, cyano, -CONHR², -NHCOR² and/or aryl- or hetarylazo, each of which may be substituted by C₁-C₁₀-alkyl, C₁-C₆-alkoxy and/or cyano;
R' is C₂-C₃₀-alkyl, whose carbon chain may be interrupted by one or more -O- and/or -CO- groups and which may be monosubstituted or polysubstituted by cyano, C₁-C₆-alkoxy, C₅-C₈-cycloalkyl, whose carbon skeleton may be interrupted by one or more -O-, -S- and/or -NR¹- groups and which may be C₁-C₆-alkyl-substituted, aryl, which may be substituted by C₁-C₁₈-alkyl or C₁-C₆-alkoxy, and/or a 5- to 7-membered heterocyclic radical which is bonded via a nitrogen atom and which may contain further heteroatoms and which may be aromatic;
methyl, which is monosubstituted or disubstituted by aryl, hetaryl and/or C₅-C₈-cycloalkyl, each of which may be substituted by C₁-C₁₈-alkyl or C₁-C₆-alkoxy;
C₅-C₈-cycloalkyl, whose carbon skeleton may be interrupted by one or more -O-, -S- and/or -NR¹- groups and which may be monosubstituted or polysubstituted by C₁-C₆-alkyl;
R¹ is hydrogen or C₁-C₆-alkyl;
R² is hydrogen; C₁-C₁₈-alkyl; aryl or hetaryl, each of which may be substituted by C₁-C₆-alkyl, C₁-C₆-alkoxy and/or cyano;
n is 2 or 3.

2. A rylene dye of the formula I as claimed in claim 1, where
R is hydrogen;
C₁-C₃₀-alkyl, whose carbon chain may be interrupted by one or more -O- and/or -CO- groups which may be monosubstituted or polysubstituted by cyano,
C₁-C₆-alkoxy, aryl, which may be substituted by C₁-C₁₈-alkyl or C₁-C₆-alkoxy, and/or a 5- to 7-membered heterocyclic radical which is bonded via a nitrogen atom and may contain further heteroatoms and may be aromatic;
aryl or hetaryl, each of which may be monosubstituted or polysubstituted by C₁-C₁₈-alkyl, C₁-C₆-alkoxy, cyano, -CONHR² or -NHCOR²;
R' is C₂-C₃₀-alkyl, whose carbon chain may be interrupted by one or more -O- and/or -CO- groups and which may be monosubstituted or polysubstituted by C₁-C₆-alkoxy, C₅-C₈-cycloalkyl, whose carbon skeleton may be interrupted by one or more -O- and/or -NR¹- groups and which may be C₁-C₆-alkyl-substituted, and/or aryl, which may be substituted by C₁-C₁₈-alkyl or C₁-C₆-alkoxy;
methyl, which is monosubstituted or disubstituted by aryl and/or C₅-C₈-cycloalkyl, each of which may be substituted by C₁-C₁₈-alkyl or C₁-C₆-alkoxy;
C₅-C₈-cycloalkyl, whose carbon skeleton may be interrupted by one or more -O- and/or -NR¹- groups and which may be monosubstituted or polysubstituted by C₁-C₆-alkyl.

3. A rylene dye of the formula I as claimed in claim 1, where
R is hydrogen;
C₁-C₃₀-alkyl, whose carbon chain may be interrupted by one or more -O- and/or -CO- groups which may be monosubstituted or disubstituted by C₁-C₆-alkoxy and/or aryl, which may be substituted by C₁-C₁₈-alkyl or C₁-C₆-alkoxy;
aryl, which may be monosubstituted or polysubstituted by C₁-C₁₈-alkyl, C₁-C₆-alkoxy and/or cyano;
R' is C₂-C₃₀-alkyl, whose carbon chain may be interrupted by one or more -O- groups and which may be monosubstituted or polysubstituted by C₁-C₆-alkoxy, C₅-C₈-cycloalkyl which may be C₁-C₆-alkyl-substituted, and/or aryl, which may be substituted by C₁-C₁₈-alkyl or C₁-C₆-alkoxy;
methyl, which is monosubstituted or disubstituted by aryl and/or C₅-C₈-cycloalkyl, each of which may be substituted by C₁-C₁₈-alkyl or C₁-C₆-alkoxy;
C₅-C₈-cycloalkyl, which may be monosubstituted or polysubstituted by C₁-C₆-alkyl.

4. A process for the preparation of a rylene dye of the formula I as claimed in claims 1 to 3, which comprises
a) reacting a bromorylene-3,4-dicarboximide of the general formula II 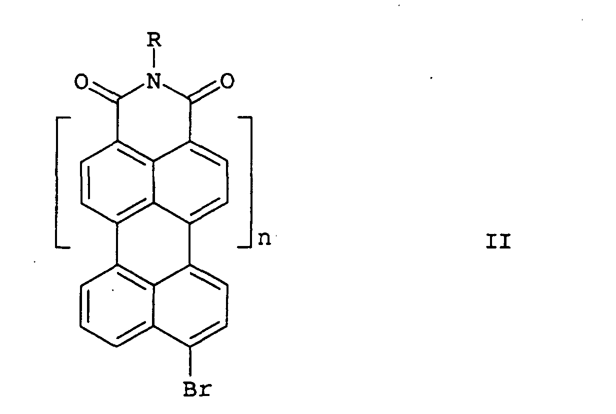 with a benzophenonimine of the general formula III 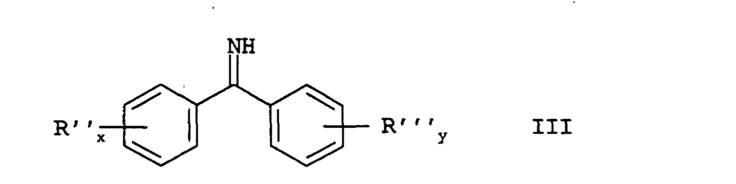 where
R'', R''', independently of one another, are hydrogen, C₁-C₆-alkyl or C₁-C₆-alkoxy and
x, y, independently of one another, are an integer from 1 to 3,
in the presence of an aprotic organic solvent, a transition-metal catalyst system and a base in an aryl-N coupling reaction,
b) hydrolysing the resultant ketimine in the presence of an acid and in the presence of a polar, aprotic solvent to give an aminorylene-3,4-dicarboximide of the general formula IV 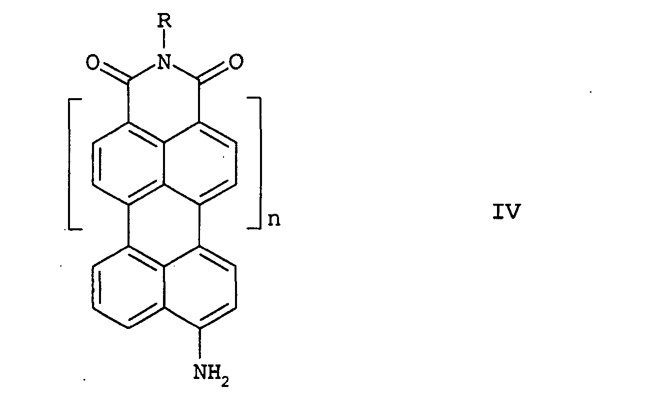 and
c) subsequently reacting the latter with a dicarbonate of the general formula V 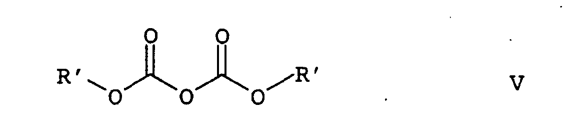 in the presence of a polar, aprotic solvent and in the presence of a base to give a rylene dye I.

5. A process for the preparation of an aminorylene-3,4-dicarboximide of the general formula IV 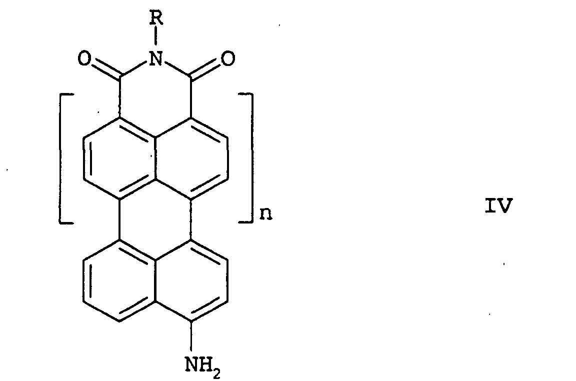 where
R is hydrogen;
C₁-C₃₀-alkyl, whose carbon chain may be interrupted by one or more -O-, -S-, -NR¹-, -CO- and/or -SO₂- groups and which may be monosubstituted or polysubstituted by cyano, C₁-C₆-alkoxy, aryl, which may be substituted by C₁-C₁₈-alkyl or C₁-C₆-alkoxy, and/or a 5- to 7-membered heterocyclic radical which is bonded via a nitrogen atom and may contain further heteroatoms and may be aromatic;
aryl or hetaryl, each of which may be monosubstituted or polysubstituted by C₁-C₁₈-alkyl, C₁-C₆-alkoxy, cyano, -CONHR², -NHCOR² and/or aryl- or hetarylazo, each of which may be substituted by C₁-C₁₀-alkyl, C₁-C₆-alkoxy and/or cyano;
R¹ is hydrogen or C₁-C₆-alkyl;
R² is hydrogen; C₁-C₁₈-alkyl; aryl or hetaryl, each of which may be substituted by C₁-C₆-alkyl, C₁-C₆-alkoxy and/or cyano;
n is 2 or 3,
which comprises
a) reacting a bromorylene-3,4-dicarboximide of the general formula II 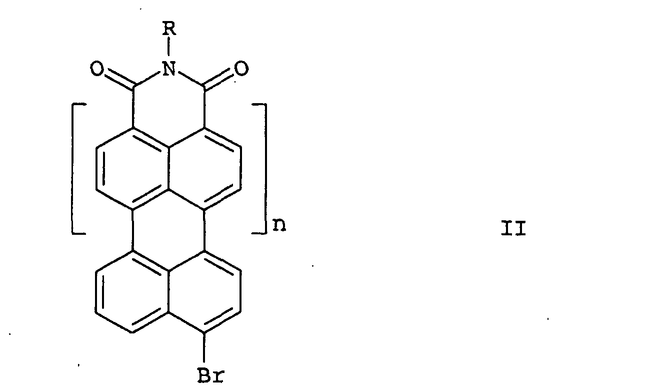 with a benzophenonimine of the general formula III 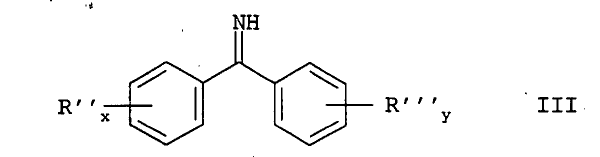 in the presence of an aprotic organic solvent, a transition-metal catalyst system and a base in an aryl-N coupling reaction, and
b) hydrolysing the resultant ketimine in the presence of an acid and in the presence of a polar, aprotic solvent.

6. An aminorylene-3,4-dicarboximide of the general formula IV 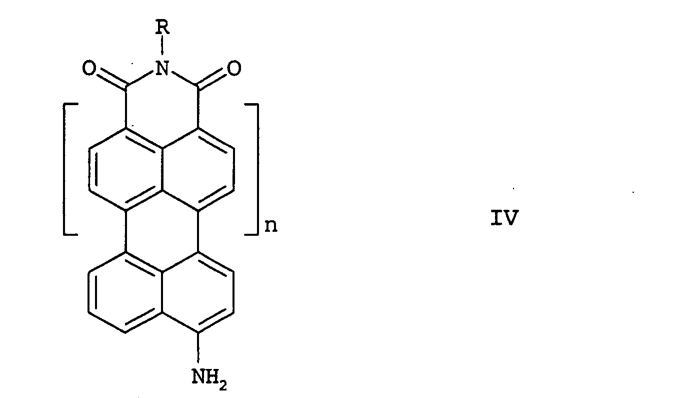 where
R is hydrogen;
C₁-C₃₀-alkyl, whose carbon chain may be interrupted by one or more -O-, -S-, -NR¹-, -CO- and/or -SO₂- groups and which may be monosubstituted or polysubstituted by cyano, C₁-C₆-alkoxy, aryl, which may be substituted by C₁-C₁₈-alkyl or C₁-C₆-alkoxy, and/or a 5- to 7-membered heterocyclic radical which is bonded via a nitrogen atom and may contain further heteroatoms and may be aromatic;
aryl or hetaryl, each of which may be monosubstituted or polysubstituted by C₁-C₁₈-alkyl, C₁-C₆-alkoxy, cyano, -CONHR², -NHCOR² and/or aryl- or hetarylazo, each of which may be substituted by C₁-C₁₀-alkyl, C₁-C₆-alkoxy and/or cyano;
R¹ is hydrogen or C₁-C₆-alkyl;
R² is hydrogen, C₁-C₁₈-alkyl; aryl or hetaryl, each of which may be substituted by C₁-C₆-alkyl, C₁-C₆-alkoxy and/or cyano;
n is 2 or 3.

7. The use of a rylene dye of the formula I as claimed in claims 1 to 3 for coloring high-molecular-weight organic and inorganic materials.

8. The use as claimed in claim 7, wherein plastics, surface coatings, printing inks and oxidic layer systems are colored.

9. The use as claimed in claim 8 or 9, wherein laser-markable and laser-inscribable colorings are produced.

## Revendications

1. Colorants rylène de formule générale I 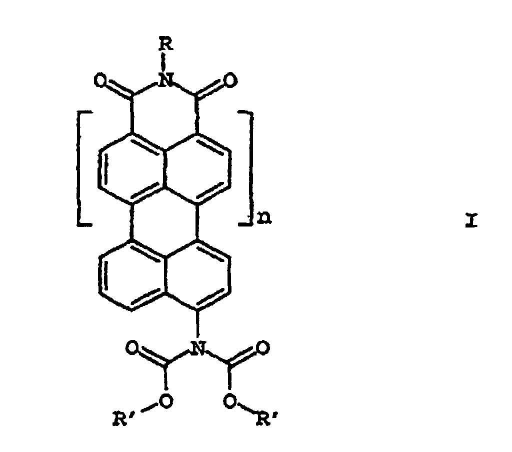 dans laquelle les variables ont la signification suivante :
R représente un atome d'hydrogène ;
un groupe alkyle en C₁ à C₃₀, dont la chaîne carbonée peut être interrompue par un ou plusieurs groupements -O-, -S-, -NR¹-, -CO- et/ou -SO₂-, et qui peut être substitué une ou plusieurs fois par un groupe cyano, alcoxy en C₁ à C₆, aryle, qui peut être substitué par un groupe alkyle en C₁ à C₁₈ ou alcoxy en C₁ à C₆, et/ou un groupe hétérocyclique de 5 à 7 éléments, lié par l'intermédiaire d'un atome d'azote, qui peut contenir d'autres hétéroatomes et qui peut être aromatique ;
un groupe aryle ou hétéroaryle, qui peut être à chaque fois substitué une ou plusieurs fois par un groupe alkyle en C₁ à C₁₈, alcoxy en C₁ à C₆, cyano, -CONHR², -NHCOR² et/ou arylazo ou hétéroarylazo, qui peut être à chaque fois substitué par un groupe alkyle en C₁ à C₁₀, alcoxy en C₁ à C₆ et/ou cyano ;
R' représente un groupe alkyle en C₂ à C₃₀, dont la chaîne carbonée peut être interrompue par un ou plusieurs groupements -O- et/ou -CO-, et qui peut être substitué une ou plusieurs fois par un groupe cyano, alcoxy en C₁ à C₆, cycloalkyle en C₅ à C₈, dont le squelette carboné peut être interrompu par un ou plusieurs groupements -O-, -S-, et/ou -NR¹- et qui peut être substitué par un groupe alkyle en C₁ à C₆, aryle, qui peut être substitué par un groupe alkyle en C₁ à C₁₈ ou alcoxy en C₁ à C₆, et/ou un groupe hétérocyclique de 5 à 7 éléments, lié par l'intermédiaire d'un atome d'azote, qui peut contenir d'autres hétéroatomes et qui peut être aromatique ;
un groupe méthyle, qui peut être substitué une ou plusieurs fois par un groupe aryle, hétéroaryle et/ou cycloalkyle en C₅ à C₈, qui peut être à chaque fois substitué par un groupe alkyle en C₁ à C₁₈ ou alcoxy en C₁ à C₆ ;
un groupe cycloalkyle en C₅ à C₈, dont le squelette carboné peut être interrompu par un ou plusieurs groupements -O-, -S- et/ou -NR¹-, et qui peut être substitué une ou plusieurs fois par un groupe alkyle en C₁ à C₆ ;
R¹ représente un atome d'hydrogène ou un groupe alkyle en C₁ à C₆ ;
R² représente un atome d'hydrogène ; un groupe alkyle en C₁ à C₁₈ ; un groupe aryle ou hétéroaryle, qui peut être à chaque fois substitué par un groupe alkyle en C₁ à C₆, alcoxy en C₁ à C₆ et/ou cyano ;
n vaut 2 ou 3.

2. Colorants rylène de formule I, selon la revendication 1, dans laquelle les variables ont la signification suivante :
R représente un atome d'hydrogène ;
un groupe alkyle en C₁ à C₃₀, dont la chaîne carbonée peut être interrompue par un ou plusieurs groupements -O- et/ou -CO- et/ou -SO₂-, et qui peut être substitué une ou plusieurs fois par un groupe cyano, alcoxy en C₁ à C₆, aryle, qui peut être substitué par un groupe alkyle en C₁ à C₁₈ ou alcoxy en C₁ à C₆, et/ou un groupe hétérocyclique de 5 à 7 éléments, lié par l'intermédiaire d'un atome d'azote, qui peut contenir d'autres hétéroatomes et qui peut être aromatique ;
un groupe aryle ou hétéroaryle, qui peut être à chaque fois substitué une ou plusieurs fois par un groupe alkyle en C₁ à C₁₈, alcoxy en C₁ à C₆, cyano, -CONHR² et/ou -NHCOR² ;
R' représente un groupe alkyle en C₂ à C₃₀, dont la chaîne carbonée peut être interrompue par un ou plusieurs groupements -O- et/ou -CO-, et qui peut être substitué une ou plusieurs fois par un groupe alcoxy en C₁ à C₆, cycloalkyle en C₅ à C₈, dont le
squelette carboné peut être interrompu par un ou plusieurs groupements -O- et/ou -NR¹- et qui peut être substitué par un groupe alkyle en C₁ à C₆, et/ou aryle, qui peut être substitué par un groupe alkyle en C₁ à C₁₈ ou alcoxy en C₁ à C₆ ;
un groupe méthyle, qui peut être substitué une ou deux fois par un groupe aryle et/ou cycloalkyle en C₅ à C₈, qui peut être à chaque fois substitué par un groupe alkyle en C₁ à C₁₈ ou alcoxy en C₁ à C₆ ;
un groupe cycloalkyle en C₅ à C₈, dont le squelette carboné peut être interrompu par un ou plusieurs groupements -O- et/ou -NR¹-, et qui peut être substitué une ou plusieurs fois par un groupe alkyle en C₁ à C₆.

3. Colorants rylène de formule I, selon la revendication 1, dans laquelle les variables ont la signification suivante :
R représente un atome d'hydrogène ;
un groupe alkyle en C₁ à C₃₀, dont la chaîne carbonée peut être interrompue par un ou plusieurs groupements -O- et/ou -CO-, et qui peut être substitué une ou deux fois par un groupe alcoxy en C₁ à C₆ et/ou aryle, qui peut être substitué par un groupe alkyle en C₁ à C₁₈ ou alcoxy en C₁ à C₆ ; un groupe aryle, qui peut être substitué une ou plusieurs fois par un groupe alkyle en C₁ à C₁₈, alcoxy en C₁ à C₆ et/ou cyano ;
R' représente un groupe alkyle en C₂ à C₃₀, dont la chaîne carbonée peut être interrompue par un ou plusieurs groupements -O-, et qui peut être substitué une ou plusieurs fois par un groupe alcoxy en C₁ à C₆, cycloalkyle en C₅ à C₈, qui peut être substitué par un groupe alkyle en C₁ à C₆, et/ou aryle, qui peut être substitué par un groupe alkyle en C₁ à C₁₈ ou alcoxy en C₁ à C₆ ;
un groupe méthyle, qui peut être substitué une ou deux fois par un groupe aryle et/ou cycloalkyle en C₅ à C₈, qui peut être à chaque fois substitué par un groupe alkyle en C₁ à C₁₈ ou alcoxy en C₁ à C₆ ; un groupe cycloalkyle en C₅ à C₈, qui peut être substitué une ou plusieurs fois par un groupe alkyle en C₁ à C₆.

4. Procédé de production de colorants rylène de formule I selon les revendications 1 à 3, **caractérisé en ce que**
a) l'on fait réagir un imide d'acide bromorylène-3,4-dicarboxylique de formule générale II 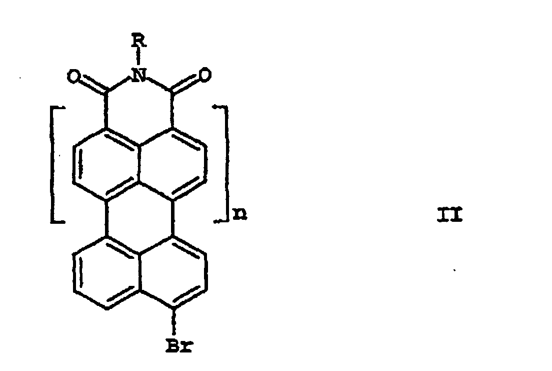 en présence d'un solvant organique aprotique, d'un système de catalyseur de métal de transition et d'une base, dans une réaction de couplage aryle-N avec une benzophénone-imine de formule générale III 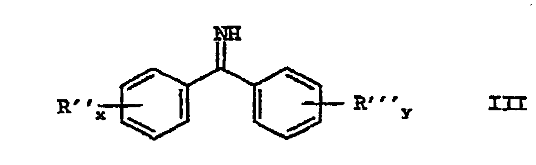 dans laquelle les variables
R'', R''' représentent, indépendamment l'un de l'autre, un atome d'hydrogène, un groupe alkyle en C₁ à C₆ ou alcoxy en C₁ à C₆ et
x, y représentent, indépendamment l'un de l'autre, un nombre entier de 1 à 3,
b) l'on hydrolyse la cétimine formée en présence d'un acide et d'un solvant polaire aprotique pour former un imide de l'acide aminorylène-3,4-dicarboxylique de formule générale IV 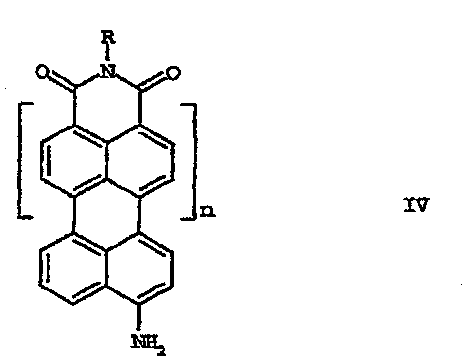
c) et on le fait ensuite réagir en présence d'un solvant polaire aprotique et d'une base avec un dicarbonate de formule générale V 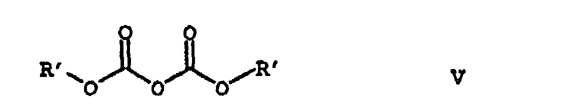 pour former le colorant rylène I.

5. Procédé de production d'imides d'acide aminorylène-3,4-dicarboxylique de formule générale IV 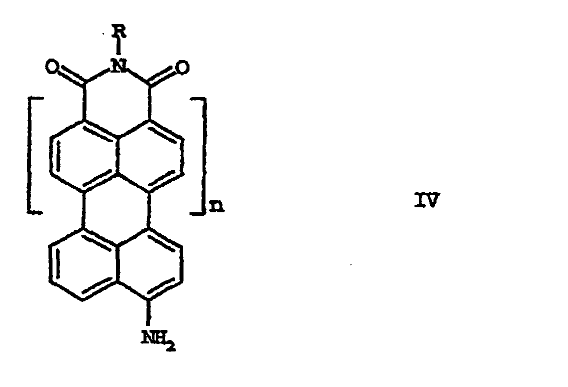 dans laquelle les variables ont la signification suivante :
R représente un atome d'hydrogène ;
un groupe alkyle en C₁ à C₃₀, dont la chaîne carbonée peut être interrompue par un ou plusieurs groupements -O-, -S-, -NR¹-, -CO- et/ou -SO₂-, et qui peut être substitué une ou plusieurs fois par un groupe cyano, alcoxy en C₁ à C₆, aryle, qui peut être substitué par un groupe alkyle en C₁ à C₁₈ ou alcoxy en C₁ à C₆, et/ou un groupe hétérocyclique de 5 à 7 éléments, lié par l'intermédiaire d'un atome d'azote, qui peut contenir d'autres hétéroatomes et qui peut être aromatique ; un groupe aryle ou hétéroaryle, qui peut être à chaque fois substitué une ou plusieurs fois par un groupe alkyle en C₁ à C₁₈, alcoxy en C₁ à C₆, cyano, -CONHR², -NHCOR² et/ou arylazo ou hétéroarylazo, qui peut être à chaque fois substitué par un groupe alkyle en C₁ à C₁₀, alcoxy en C₁ à C₆ et/ou cyano ;
R¹ représente un atome d'hydrogène ou un groupe alkyle en C₁ à C₆ ;
R² représente un atome d'hydrogène ; un groupe alkyle en C₁ à C₁₈ ; un groupe aryle ou hétéroaryle, qui peut être à chaque fois substitué par un groupe alkyle en C₁ à C₆, alcoxy en C₁ à C₆ et/ou cyano ;
n vaut 2 ou 3,
**caractérisé en ce que**
a) l'on fait réagir un imide d'acide bromorylène-3,4-dicarboxylique de formule générale II 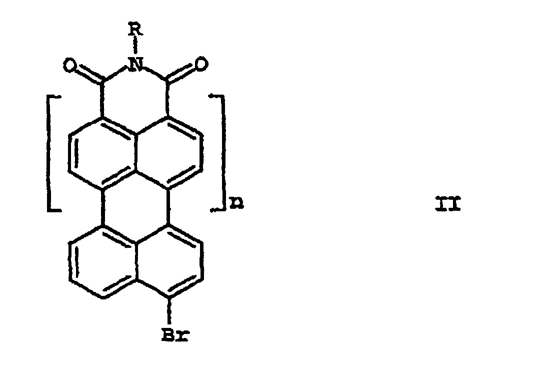 en présence d'un solvant organique aprotique, d'un système de catalyseur de métal de transition et d'une base, dans une réaction de couplage aryle-N avec une benzophénone-imine de formule générale III 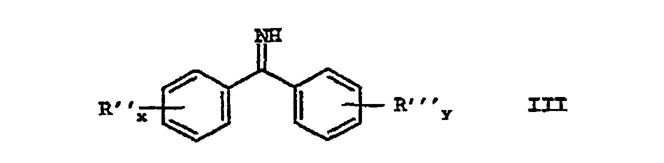
b) et que l'on hydrolyse en présence d'un acide et d'un solvant polaire aprotique la cétimine formée.

6. Imides d'acide aminorylène-3,4-dicarboxylique de formule générale IV 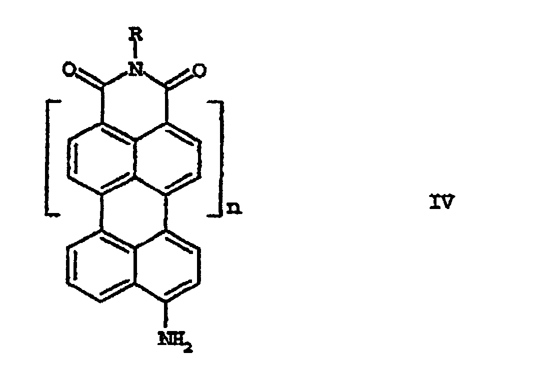 dans laquelle les variables ont la signification suivante :
R représente un atome d'hydrogène ;
un groupe alkyle en C₁ à C₃₀, dont la chaîne carbonée peut être interrompue par un ou plusieurs groupements -O-, -S-, -NR¹-, -CO- et/ou -SO₂-, et qui peut être substitué une ou plusieurs fois par un groupe cyano, alcoxy en C₁ à C₆, aryle, qui peut être substitué par un groupe alkyle en C₁ à C₁₈ ou alcoxy en C₁ à C₆, et/ou un groupe hétérocyclique de 5 à 7 éléments, lié par l'intermédiaire d'un atome d'azote, qui peut contenir d'autres hétéroatomes et qui peut être aromatique ;
un groupe aryle ou hétéroaryle, qui peut être à chaque fois substitué une ou plusieurs fois par un groupe alkyle en C₁ à C₁₈, alcoxy en C₁ à C₆, cyano, -CONHR², -NHCOR² et/ou arylazo ou hétéroarylazo, qui peut être à chaque fois substitué par un groupe alkyle en C₁ à C₁₀, alcoxy en C₁ à C₆ et/ou cyano ;
R¹ représente un atome d'hydrogène ou un groupe alkyle en C₁ à C₆ ;
R² représente un atome d'hydrogène ; un groupe alkyle en C₁ à C₁₈ ; un groupe aryle ou hétéroaryle, qui peut être à chaque fois substitué par un groupe alkyle en C₁ à C₆, alcoxy en C₁ à C₆ et/ou cyano ;
n vaut 2 ou 3.

7. Utilisation de colorants rylène de formule I selon les revendications 1 à 3 pour colorer des matières organiques et inorganiques de masse moléculaire élevée.

8. Utilisation selon la revendication 7, **caractérisée en ce que** l'on colore des matières plastiques, des vernis, des encres d'impression et des systèmes stratifiés contenant des oxydes.

9. Utilisation selon la revendication 8 ou 9, **caractérisée en ce que** l'on produit des colorations marquables au laser et imprimables au laser.
